# EUROPÄISCHE PATENTSCHRIFT

(11) **EP 0 797 573 B1**
(45) Veröffentlichungstag und Bekanntmachung des Hinweises auf die Patenterteilung: **10.04.2002**
(21) Anmeldenummer: 95942066.2
(22) Anmeldetag: 04.12.1995
(51) Int. Cl.: C07D 249/12, C07D 413/04, C07D 405/04, A01N 43/653

(54) **4-HETEROARYL-1,2,4-TRIAZOLDERIVATE MIT HERBIZIDEN EIGENSCHAFTEN**
4-HETEROARYL-1,2,4-TRIAZOLE DERIVATIVES WITH HERBICIDAL PROPERTIES
DERIVES DE 4-HETEROARYLES-1,2,4-TRIAZOLE PRESENTANT DES PROPRIETES HERBICIDES

(30) Priorität: 15.12.1994 DE 4444741; 24.08.1995 DE 19531152
(43) Veröffentlichungstag der Anmeldung: 01.10.1997
(60) Teilanmeldung: 01110136.7
(73) Patentinhaber: BAYER AG, 51368 Leverkusen (DE); NIHON BAYER AGROCHEM K.K., Tokyo 108 (JP)
(72) Erfinder: LINKER, Karl-Heinz, D-51377 Leverkusen (DE); FINDEISEN, Kurt, D-51375 Leverkusen (DE); SCHALLNER, Otto, D-40789 Monheim (DE); LENDER, Andreas, D-21376 Salzhausen (DE); SANTEL, Hans-Joachim, D-51371 Leverkusen (DE); DOLLINGER, Markus, D-51381 Leverkusen (DE); YANAGI, Akihiko, Tochigi (JP); GOTO, Toshio, Tochigi (JP)
(74) Vertreter: Linkenheil, Dieter, Dr.
(86) Internationale Anmeldenummer: EP9504759
(87) Internationale Veröffentlichungsnummer: WO9618618

(56) Entgegenhaltungen:
- EP-A- 0 011 693
- GB-A- 2 041 353
- GB-A- 2 055 826
- GB-A- 2 063 855
- US-A- 5 108 486
- JOURNAL OF PHARMACEUTICAL SCIENCES, Bd. 82, Nr. 4, April 1993 WASHINGTON DC, US, Seiten 408-15, R. SIMLOT ET AL. 'Synthesis and hypolipidemic activity of 4-substituted 1-acyl-1,2,4-triazolidine-3,5-diones in rodents'
- CHEMICAL ABSTRACTS, vol. 89, no. 3, 17.Juli 1978 Columbus, Ohio, US; abstract no. 24319z, Seite 653; & JP,A,78 018 571 (MITSUBISHI CHEMICAL INDUSTRIES CO., LTD.) 20.Februar 1978

## Beschreibung

Die Erfindung betrifft neue substituierte N-Aryl-Stickstoffheterocyclen, Verfahren zu ihrer Herstellung und ihre Verwendung als Herbizide.

Es ist bekannt, daß bestimmte N-Aryl-Stickstoffheterocyclen herbizide Eigenschaften aufweisen (vgl. EP 11693, DE 2952685, DE 3026739, US 4276420, US 4326878, WO 94/14817, EP 75267 und EP 210137 GB-A-2 063 855, US-A-5 108 486). Die aus den angegebenen Patentanmeldungen bekannten Verbindungen haben jedoch keine nennenswerte Bedeutung erlangt.

Es wurden nun die neuen substituierten N-Aryl-Stickstoffheterocyclen der allgemeinen Formel (I) gefunden, 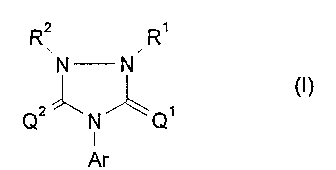 in welcher
- Q¹: für Sauerstoff oder Schwefel steht,
- Q²: für Sauerstoff oder Schwefel steht,
- R¹: für Wasserstoff, Cyano, Formyl oder für jeweils gegebenenfalls durch Fluor, Chlor, Cyano, Carboxy, C₁-C₄-Alkoxy, C₃-C₄-Alkenyloxy, C₃-C₄-Alkinyloxy, C₁-C₄-Alkylthio, C₃-C₄-Alkenylthio, C₃-C₄-Alkinylthio, C₁-C₄-Alkoxy-carbonyl, C₃-C₄-Alkenyloxy-carbonyl oder C₃-C₄-Alkinyloxy-carbonyl substituiertes C₁-C₆-Alkyl steht,
- R¹: weiterhin für jeweils gegebenenfalls durch Fluor oder Chlor substituiertes C₃-C₆-Alkenyl oder C₃-C₆-Alkinyl steht,
- R¹: weiterhin für jeweils gegebenenfalls durch Fluor oder Chlor substituiertes C₁-C₆-Alkyl-carbonyl, C₃-C₆-Alkenyl-carbonyl, C₃-C₆-Alkinyl-carbonyl, C₁-C₆-Alkoxy-carbonyl, C₃-C₆-Alkenyloxy-carbonyl oder C₃-C₆-Alkinyloxycarbonyl steht,
- R¹: weiterhin für jeweils gegebenenfalls durch Fluor, Chlor, Brom, Cyano oder Carboxy substituiertes C₃-C₆-Cycloalkyl oder C₃-C₆-Cycloalkyl-carbonyl steht,
- R²: für Wasserstoff, Cyano, Formyl oder für jeweils gegebenenfalls durch Fluor, Chlor, Cyano, Carboxy, C₁-C₄-Alkoxy, C₃-C₄-Alkenyloxy, C₃-C₄-Alkinyloxy, C₁-C₄-Alkylthio, C₃-C₄-Alkenylthio, C₃-C₄-Alkinylthio, C₁-C₄-Alkoxy-carbonyl, C₃-C₄-Alkenyloxy-carbonyl oder C₃-C₄-Alkinyloxycarbonyl substituiertes C₁-C₆-Alkyl steht,
- R²: weiterhin für jeweils gegebenenfalls durch Fluor oder Chlor substituiertes C₃-C₆-Alkenyl oder C₃-C₆-Alkinyl steht,
- R²: weiterhin für jeweils gegebenenfalls durch Fluor oder Chlor substituiertes C₁-C₆-Alkyl-carbonyl, C₃-C₆-Alkenyl-carbonyl, C₃-C₆-Alkinyl-carbonyl, C₁-C₆-Alkoxy-carbonyl, C₃-C₆-Alkenyloxy-carbonyl oder C₃-C₆-Alkinyloxycarbonyl steht,
- R²: weiterhin für jeweils gegebenenfalls durch Fluor, Chlor, Brom, Cyano oder Carboxy substituiertes C₃-C₆-Cycloalkyl oder C₃-C₆-Cycloalkyl-carbonyl steht, und
- Ar: für die nachstehend definierte substituierte, bicyclische Heteroaryl-Gruppierung steht, 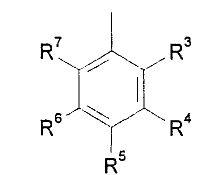 worin
R³ für Wasserstoff, Fluor, Chlor oder Brom steht,
R⁴ für Wasserstoff, Fluor, Chlor oder Brom steht,
R⁷ für Wasserstoff, Fluor oder Chlor steht,
R⁵ und R⁶ - zusammen für eine der nachstehenden Gruppierungen stehen
-Q³-C(R⁸,R⁹)-Q⁵-,
Q³-C(R⁸,R⁹)-C(R⁸,R⁹)-Q⁵-,
-Q³-CQ⁴-N(R¹⁰)-, -Q³-C(R⁸,R⁹)-CQ⁴-N(R¹⁰)-,
-C(R⁸,R⁹)-C(R⁸,R⁹)-CQ⁴-N(R¹⁰)-,
-C(R⁸)=C(R⁸)-CQ⁴-N(R¹⁰)-, -C(R⁸,R⁹)-CQ⁴-N(R¹⁰)-,
-N(R¹⁰)-C(R⁸,R⁹)-CQ⁴-N(R¹⁰)-,
wobei
- Q³, Q⁴ und Q⁵: gleich oder verschieden sind und jeweils für Sauerstoff oder Schwefel stehen,
- R⁸ und R⁹: gleich oder verschieden sind und einzeln für Wasserstoff, Fluor, Chlor, Brom oder C₁-C₄-Alkyl stehen , und
- R¹⁰: für Wasserstoff, Hydroxy oder für gegebenenfalls durch Cyano, Fluor, Chlor, C₁-C₄-Alkoxy, C₁-C₄-Alkyl-carbonyl oder C₁-C₄-Alkoxy-carbonyl substituiertes Alkyl, Alkylcarbonyl, Alkoxycarbonyl oder Alkylsulfonyl mit jeweils 1 bis 6 Kohlenstoffatomen in den Alkylgruppen steht,
- R¹⁰: weiterhin für jeweils gegebenenfalls durch Fluor, Chlor oder Brom substituiertes Alkenyl oder Alkinyl mit jeweils 2 bis 6 Kohlenstoffatomen steht,
- R¹⁰: weiterhin für jeweils gegebenenfalls durch Fluor, Chlor, Brom oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 3 Atomen in der Alkylgruppe steht,
- R¹⁰: weiterhin für jeweils gegebenenfalls durch Fluor und/oder Chlor substituiertes Alkoxy oder Alkenyloxy mit jeweils bis zu 6 Kohlenstoffatomen steht,
- R¹⁰: weiterhin für jeweils gegebenenfalls durch Cyano, Fluor, Chlor, C₁-C₄-Alkyl, C₁-C₄-Halogenalkyl, C₁-C₄-Alkoxy oder C₁-C₄-Halogenalkoxy substituiertes Benzyl oder Benzyloxy steht.

Es wurden ferner auch die zu den substituierten N-Aryl-Stickstoffheterocyclen der Formel (I) isomeren Verbindungen der Formeln (Ia) und (Ib) gefunden, 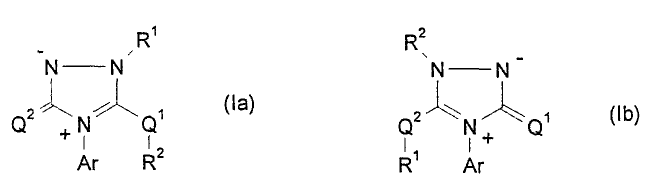 in welcher
- Q¹, Q², R¹, R² und Ar: die oben angegebenen Bedeutungen haben.

Diese Verbindungen sind jedoch nicht Gegenstand der vorliegenden Erfindung Man erhält die neuen substituierten N-Aryl-Stickstoffheterocyclen der allgemeinen Formel (I), wenn man
(a) (Thio)Semicarbazid-Derivate der allgemeinen Formel (II) 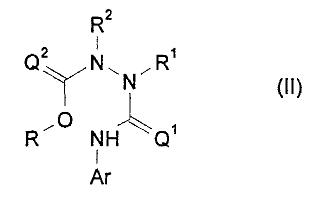 in welcher
   - Q¹, Q², R¹, R² und Ar: die oben angegebenen Bedeutungen haben und
   - R: für Alkyl steht,
   gegebenenfalls in Gegenwart eines Reaktionshilfsmittels und gegebenenfalls in Gegenwart eines Verdünnungsmittels cyclisierend kondensiert und gegebenenfalls im Anschluß daran im Rahmen der Substituentendefinition auf übliche Weise elektrophile oder nucleophile Substitutionsreaktionen durchführt,
   oder wenn man
(b) Aryliminoheterocyclen der allgemeinen Formel (III) 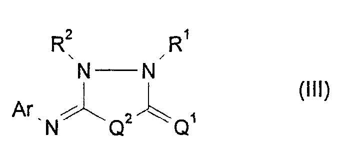 in welcher
   - Q¹, Q², R¹, R² und Ar: die oben angegebenen Bedeutungen haben,
   gegebenenfalls in Gegenwart eines Reaktionshilfsmittels und gegebenenfalls in Gegenwart eines Verdünnungsmittels thermisch ("pyrolytisch") isomerisiert.

Die Verbindungen der Formel (I) können prinzipiell auch wie im Folgenden schematisch dargestellt synthetisiert werden:
(c) Umsetzung von Aryliso(thio)cyanaten der Formel (IV) mit Hydrazinen der Formel (V) zu Aryl(thio)semicarbaziden der Formel (VI) und deren Umsetzung mit (Thio)Phosgen: 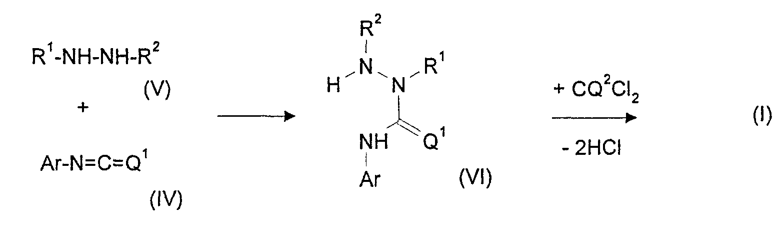
(d) Umsetzung von Aryliso(thio)cyanaten der Formel (IV) mit S-Alkyldithiocarbazaten der Formel (VII) und anschließende cyclisierende Kondensation: 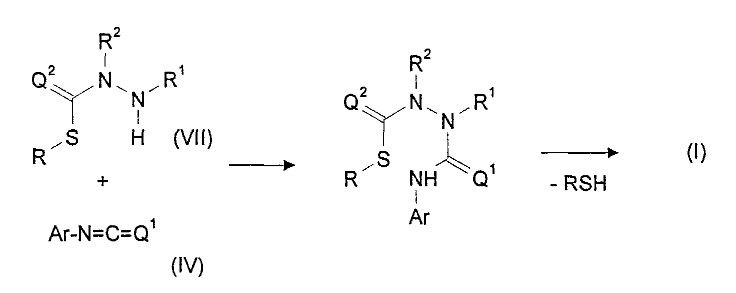
(e) Umsetzung von N,N-Bis-chlorcarbonyl- oder N,N-Bis-phenoxycarbonyl-arylaminen der Formel (VIII) - Y: Cl oder OC₆H₅ - mit Hydrazinen der Formel (V): 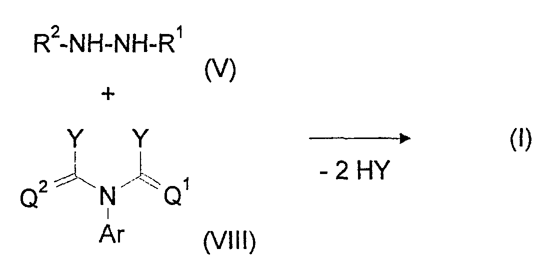
(f) Umsetzung von Arylaminen der Formel (IX) mit Hydrazindicarbonsäureestem der Formel (X):

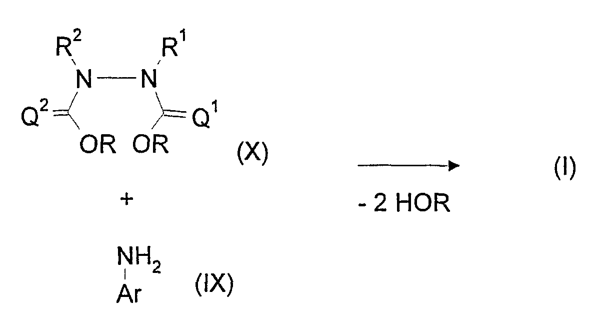

Die Verbindungen der allgemeinen Formel (I) können auch nach weiteren üblichen Methoden in andere Verbindungen der allgemeinen Formel (I) gemäß obiger Definition umgewandelt werden, beispielsweise durch übliche Umwandlungen von Carbonsäure-Gruppierungen oder deren Derivaten (z.B. R⁵: COOH → COCl, COOH → COOCH3, COCl → CONH₂, COOCH₃ → CONH₂, CONH₂ → CN, CN → CSNH₂), durch Alkylierungsreaktionen (z.B. R¹: H → CH₃ oder CHF₂) oder durch Oxidation oder Schwefelung (z.B. Q¹: O → S oder S → O) - vgl. auch die Herstellungsbeispiele.

Die neuen substituierten N-Aryl-Stickstoffheterocyclen der allgemeinen Formel (I) zeichnen sich durch starke herbizide Wirksamkeit aus.

In den Definitionen sind die gesättigten oder ungesättigten Kohlenwasserstoffketten, wie Alkyl, Alkenyl oder Alkinyl jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I), in welcher
- Q¹: für Sauerstoff oder Schwefel steht,
- Q²: für Sauerstoff oder Schwefel steht,
- R¹: für Wasserstoff, Cyano, Formyl oder für jeweils gegebenenfalls durch Fluor, Chlor, Cyano, Carboxy, Methoxy oder Ethoxy substituiertes Methyl, Ethyl, n-oder i-Propyl, n-, i-, s- oder t-Butyl steht,
- R¹: weiterhin für jeweils gegebenenfalls durch Fluor oder Chlor substituiertes Propenyl, Butenyl, Propinyl oder Butinyl steht,
- R¹: weiterhin für jeweils gegebenenfalls durch Fluor oder Chlor substituiertes Acetyl, Propionyl, Methoxycarbonyl oder Ethoxycarbonyl steht,
- R¹: weiterhin für gegebenenfalls durch Fluor oder Chlor substituiertes Cyclopropyl steht,
- R²: für Wasserstoff, Cyano, Formyl oder für jeweils gegebenenfalls durch Fluor, Chlor, Cyano, Carboxy, Methoxy oder Ethoxy substituiertes Methyl, Ethyl, n-oder i-Propyl, n-, i-, s- oder t-Butyl steht,
- R²: weiterhin für jeweils gegebenenfalls durch Fluor oder Chlor substituiertes Propenyl, Butenyl, Propinyl oder Butinyl steht,
- R²: weiterhin für jeweils gegebenenfalls durch Fluor oder Chlor substituiertes Acetyl, Propionyl, Methoxycarbonyl oder Ethoxycarbonyl steht,
- R²: weiterhin für gegebenenfalls durch Fluor oder Chlor substituiertes Cyclopropyl steht, und
- Ar: für die nachstehend definierte substituierte, bicyclische Heteroaryl-Gruppierung steht, 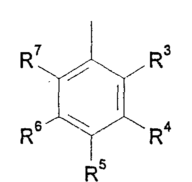 worin
R³ für Wasserstoff, Fluor oder Chlor steht,
R⁴ für Wasserstoff, Fluor oder Chlor oder steht,
R⁷ für Wasserstoff, Fluor oder Chlor steht,
R⁵ und R⁶ - zusammen für eine der nachstehenden Gruppierungen stehen
-Q³-C(R⁸,R⁹)-Q⁵-,
-Q³-C(R⁸,R⁹)-C(R⁸,R⁹)-Q⁵-,
-Q³-CQ⁴-N(R¹⁰)-,
-Q³-C(R⁸,R⁹)-CQ⁴-N(R¹⁰)-,
-C(R⁸,R⁹)-C(R⁸,R⁹)-CQ⁴-N(R¹⁰)-,
-C(R⁸)=C(R⁸)-CQ⁴-N(R¹⁰)-,
-C(R⁸,R⁹)-CQ⁴-N(R¹⁰)-, -N(R¹⁰)-C(R⁸;R⁹)-CQ⁴-N(R¹⁰)-,
wobei
- Q³, Q⁴ und Q⁵: gleich oder verschieden sind und jeweils für Sauerstoff oder Schwefel stehen,
- R⁸ und R⁹: gleich oder verschieden sind und einzeln fiir Wasserstoff, Fluor, Chlor, Methyl oder Ethyl stehen, und
- R¹⁰: für Wasserstoff, Hydroxy oder für gegebenenfalls durch Cyano, Fluor, Chlor, Methoxy, Ethoxy, Acetyl, Propionyl, Methoxycarbonyl oder Ethoxy-carbonyl substituiertes Methyl, Ethyl, n- oder i-Propyl, n-, i-,s oder t-Butyl steht,
- R¹⁰: weiterhin für jeweils gegebenenfalls durch Fluor, Chlor oder Brom substituiertes Propenyl, Butenyl, Propinyl oder Butinyl steht,
- R¹⁰: weiterhin für jeweils gegebenenfalls durch Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
- R¹⁰: weiterhin für jeweils gegebenenfalls durch Fluor und/oder Chlor substituiertes Methoxy, Ethoxy, n- oder i-Propoxy, n-, i- oder s-Butoxy, Propenyloxy oder Butenyloxy steht,
- R¹⁰: weiterhin für jeweils gegebenenfalls durch Cyano, Fluor, Chlor, Methyl, Ethyl, Trifluormethyl, Methoxy, Ethoxy, Difluormethoxy oder Trifluormethoxy substituiertes Benzyl oder Benzyloxy steht.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangstoffe bzw. Zwischenprodukte. Diese Restedefinitionen können untereinander, also auch zwischen den angegebenen Bereichen bevorzugter Verbindungen, beliebig kombiniert werden.

Beispiele für die erfindungsgemäßen Verbindungen der Formel (I) sind in den nachstehenden Gruppen aufgeführt.

### Gruppe 1

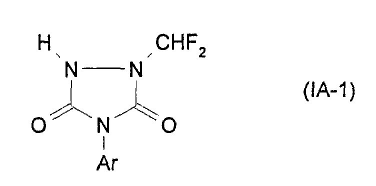

Ar hat hierbei beispielhaft die im Folgenden aufgeführten Bedeutungen:

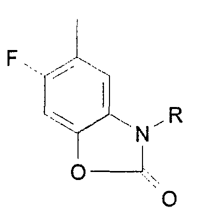

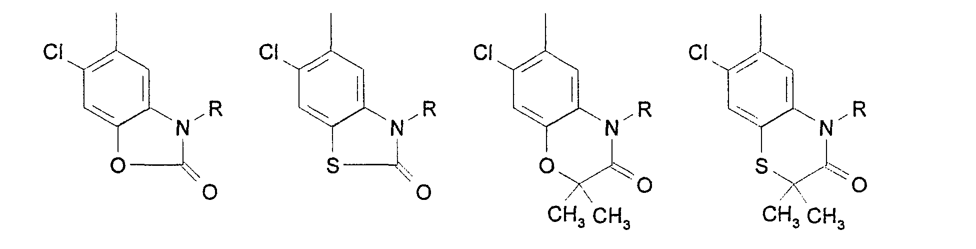

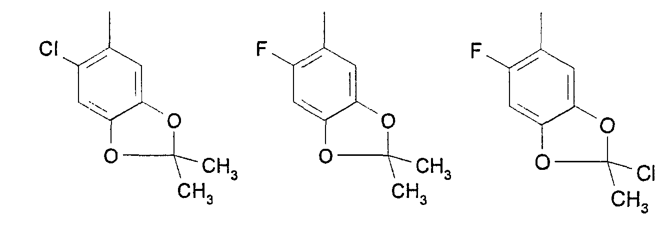

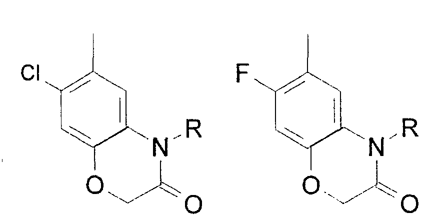

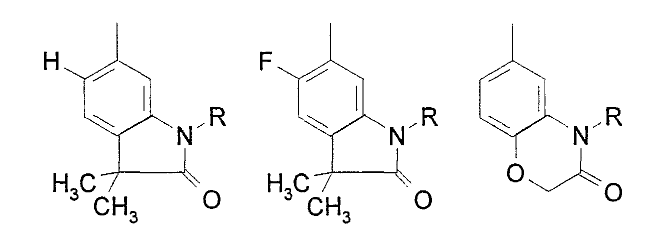

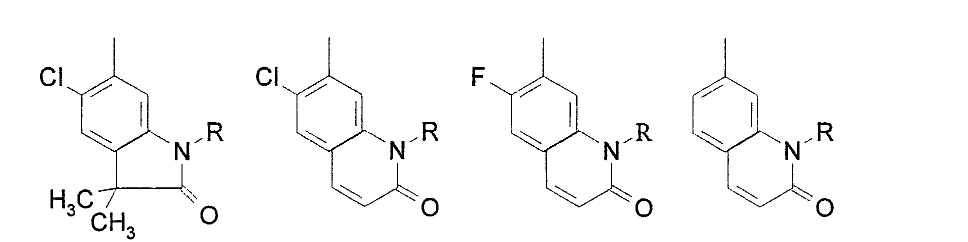

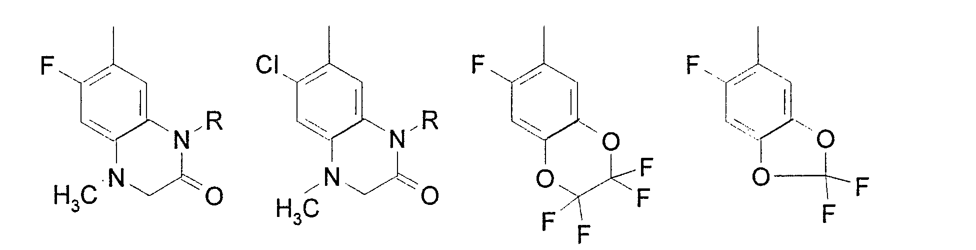

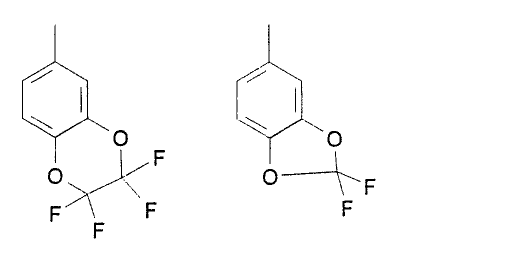

R steht hierbei beispielsweise für Wasserstoff, Hydroxy, Methyl, Ethyl, n- oder i-Propyl, n-, i- oder s-Butyl, Allyl, Propargyl, Methoxy, Ethoxy, n- oder i-Propoxy, Cyanomethyl, Carboxymethyl, Methoxymethyl, Ethoxymethyl, Methoxycarbonylmethyl, Ethoxycarbonylmethyl, Acetyl, Propionyl, Methoxycarbonyl, Ethoxycarbonyl, Methylsulfonyl oder Ethylsulfonyl.

### Gruppe 2

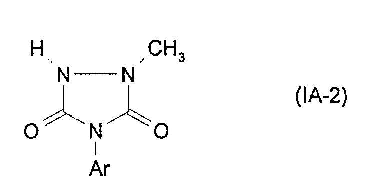

Ar hat hierbei beispielhaft die oben in Gruppe 1 aufgeführten Bedeutungen.

### Gruppe 3

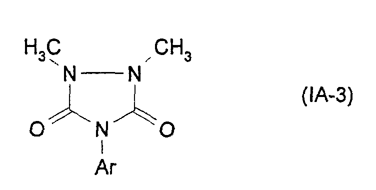

Ar hat hierbei beispielhaft die oben in Gruppe 1 aufgeführten Bedeutungen.

### Gruppe 4

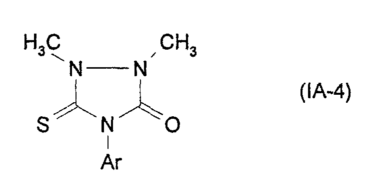

Ar hat hierbei beispielhaft die oben in Gruppe 1 aufgeführten Bedeutungen.

### Gruppe 5

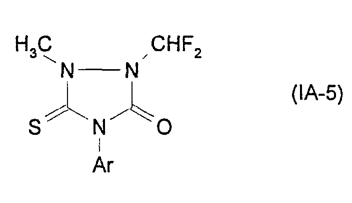

Ar hat hierbei beispielhaft die oben in Gruppe 1 aufgeführten Bedeutungen.

### Gruppe 6

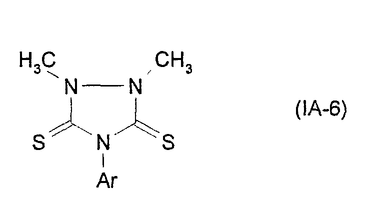

Ar hat hierbei beispielhaft die oben in Gruppe 1 aufgeführten Bedeutungen.

### Gruppe 7

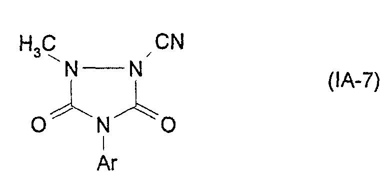

Ar hat hierbei beispielhaft die oben in Gruppe 1 aufgeführten Bedeutungen.

### Gruppe 8

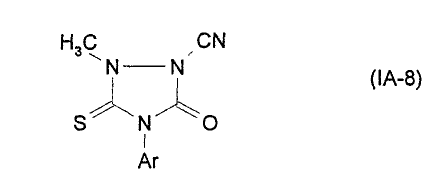

Ar hat hierbei beispielhaft die oben in Gruppe 1 aufgeführten Bedeutungen.

### Gruppe 9

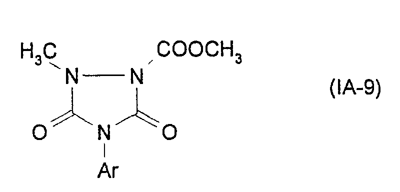

Ar hat hierbei beispielhaft die oben in Gruppe 1 aufgeführten Bedeutungen.

### Gruppe 10

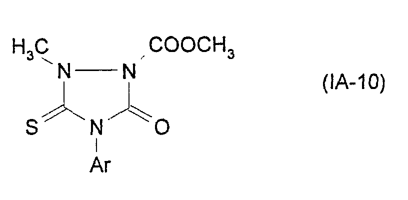

Ar hat hierbei beispielhaft die oben in Gruppe 1 aufgeführten Bedeutungen.

### Gruppe 11

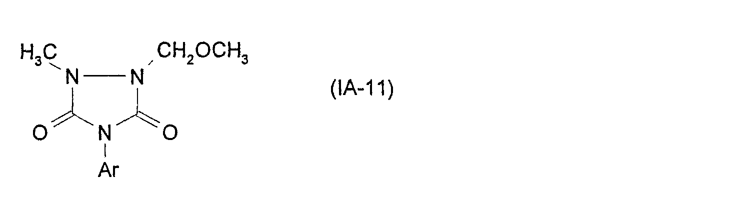

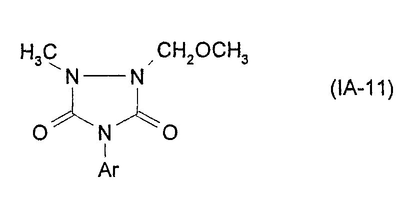

Ar hat hierbei beispielhaft die oben in Gruppe 1 aufgeführten Bedeutungen.

### Gruppe 12

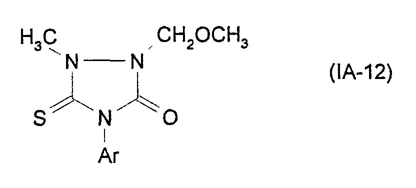

Ar hat hierbei beispielhaft die oben in Gruppe 1 aufgeführten Bedeutungen.

### Gruppe 13

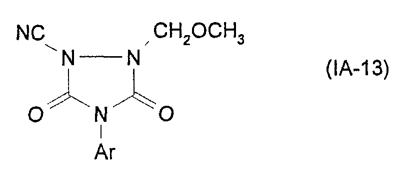

Ar hat hierbei beispielhaft die oben in Gruppe 1 aufgeführten Bedeutungen.

### Gruppe 14

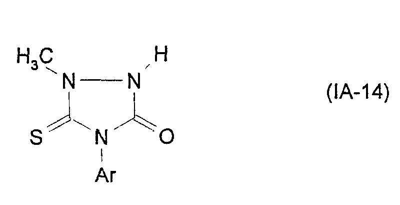

Ar hat hierbei beispielhaft die oben in Gruppe 1 aufgeführten Bedeutungen.

### Gruppe 15

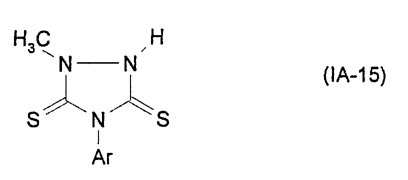

Ar hat hierbei beispielhaft die oben in Gruppe 1 aufgeführten Bedeutungen.

### Gruppe 16

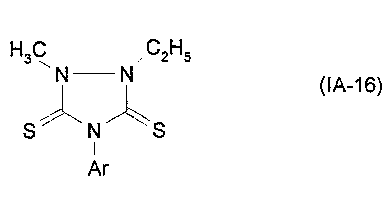

Ar hat hierbei beispielhaft die oben in Gruppe 1 aufgeführten Bedeutungen.

### Gruppe 17

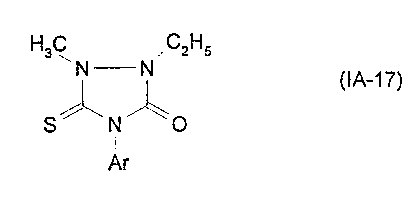

Ar hat hierbei beispielhaft die oben in Gruppe 1 aufgeführten Bedeutungen.

### Gruppe 18

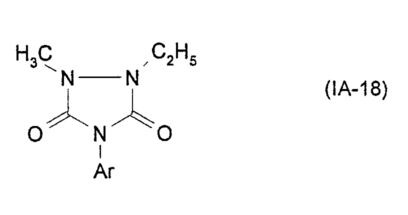

Ar hat hierbei beispielhaft die oben in Gruppe 1 aufgeführten Bedeutungen.

### Gruppe 19

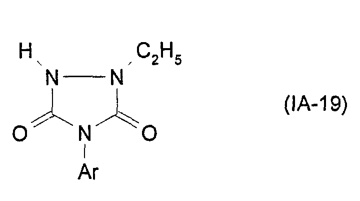

Ar hat hierbei beispielhaft die oben in Gruppe 1 aufgeführten Bedeutungen.

### Gruppe 20

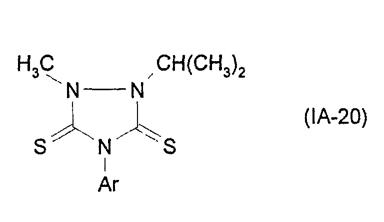

Ar hat hierbei beispielhaft die oben in Gruppe 1 aufgeführten Bedeutungen.

### Gruppe 21

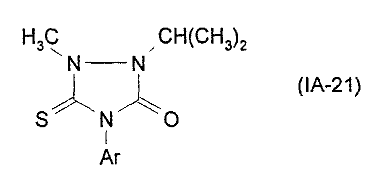

Ar hat hierbei beispielhaft die oben in Gruppe 1 aufgeführten Bedeutungen.

### Gruppe 22

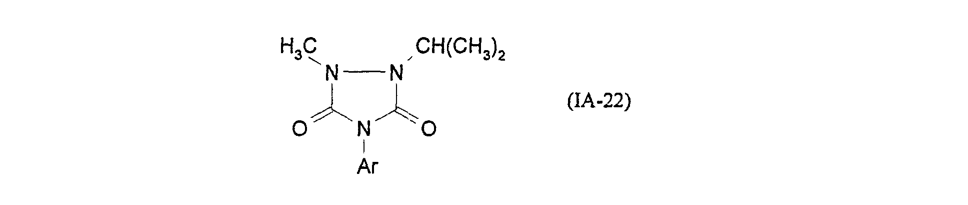

Ar hat hierbei beispielhaft die oben in Gruppe 1 aufgeführten Bedeutungen.

### Gruppe 23

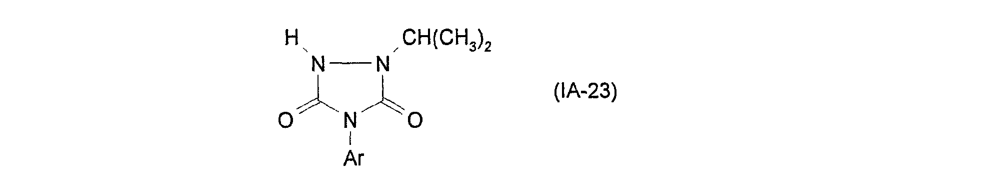

Ar hat hierbei beispielhaft die oben in Gruppe 1 aufgeführten Bedeutungen.

### Gruppe 24

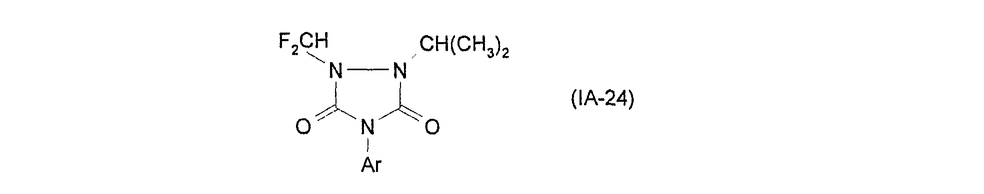

Ar hat hierbei beispielhaft die oben in Gruppe 1 aufgeführten Bedeutungen.

### Gruppe 25

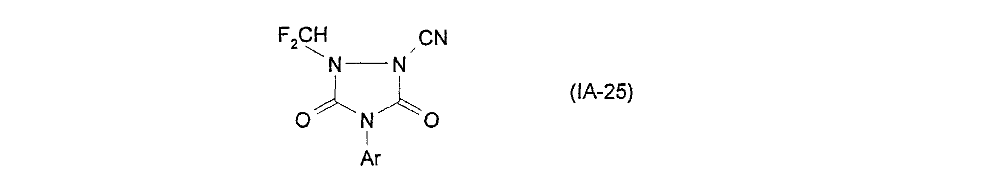

Ar hat hierbei beispielhaft die oben in Gruppe 1 aufgeführten Bedeutungen.

### Gruppe 26

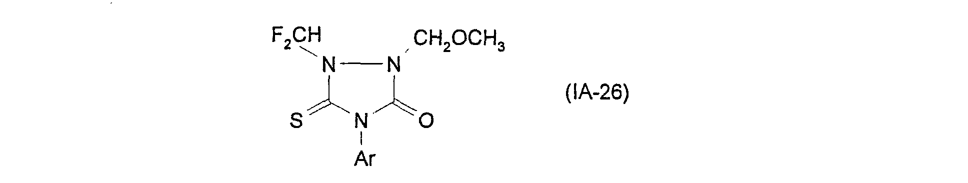

Ar hat hierbei beispielhaft die oben in Gruppe 1 aufgeführten Bedeutungen.

### Gruppe 27

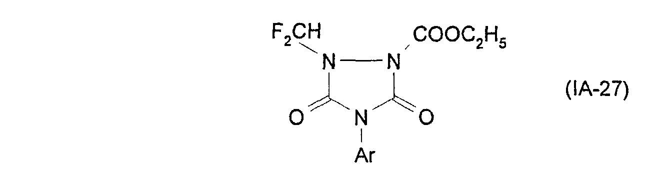

Ar hat hierbei beispielhaft die oben in Gruppe 1 aufgeführten Bedeutungen.

### Gruppe 28

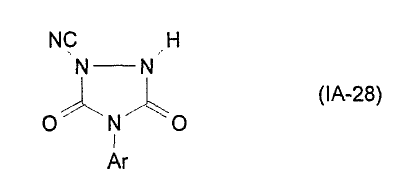

Ar hat hierbei beispielhaft die oben in Gruppe 1 aufgeführten Bedeutungen.

### Gruppe 29

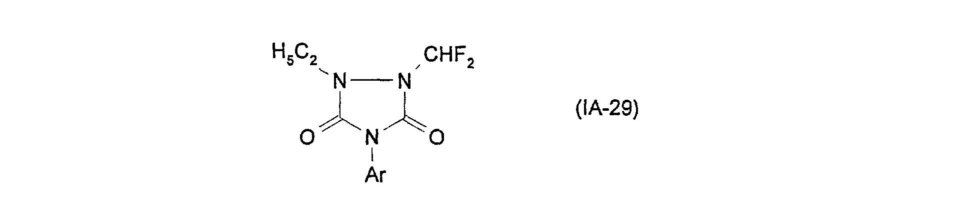

Ar hat hierbei beispielhaft die oben in Gruppe 1 aufgeführten Bedeutungen.

### Gruppe 30

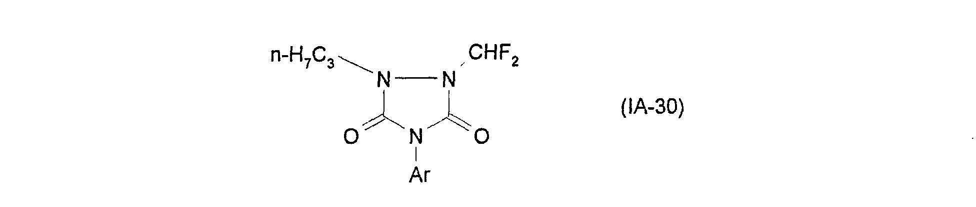

Ar hat hierbei beispielhaft die oben in Gruppe 1 aufgeführten Bedeutungen.

### Gruppe 31

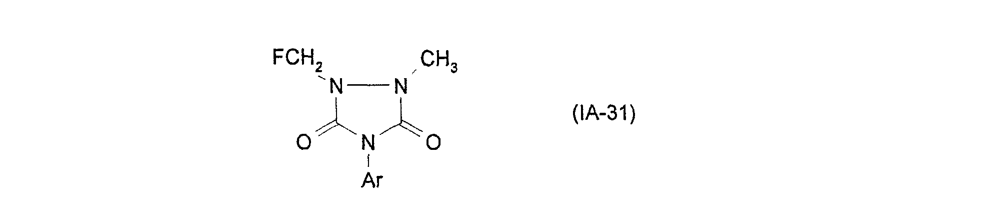

Ar hat hierbei beispielhaft die oben in Gruppe 1 aufgeführten Bedeutungen.

### Gruppe 32

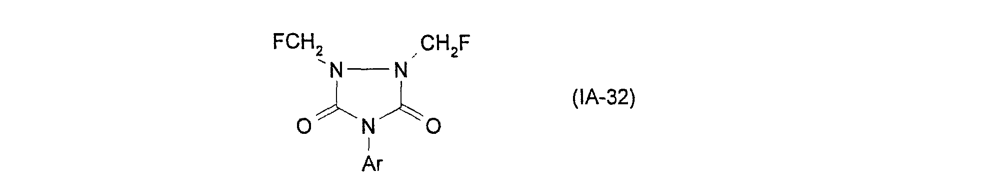

Ar hat hierbei beispielhaft die oben in Gruppe 1 aufgeführten Bedeutungen.

### Gruppe 33

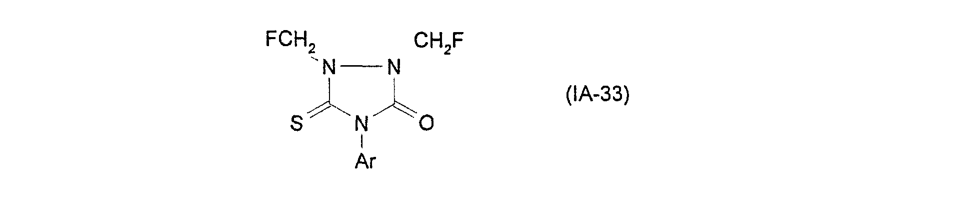

Ar hat hierbei beispielhaft die oben in Gruppe 1 aufgeführten Bedeutungen.

### Gruppe 34

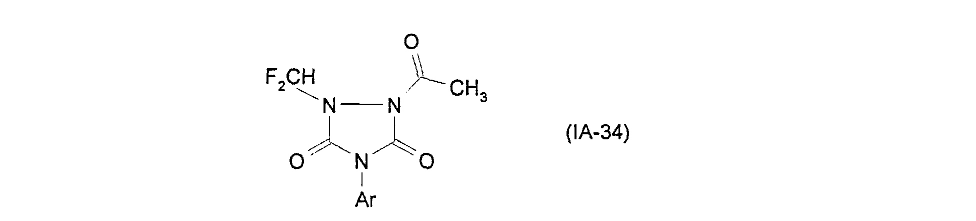

Ar hat hierbei beispielhaft die oben in Gruppe 1 aufgeführten Bedeutungen.

### Gruppe 35

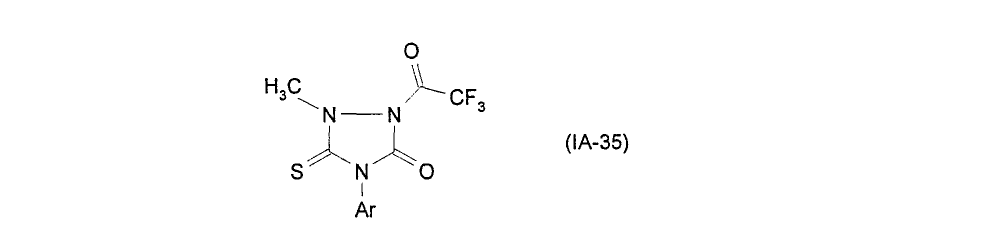

Ar hat hierbei beispielhaft die oben in Gruppe 1 aufgeführten Bedeutungen.

### Gruppe 36

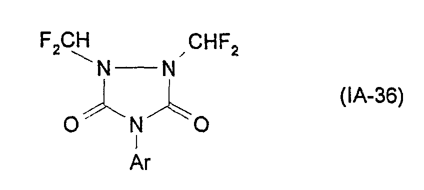

Ar hat hierbei beispielhaft die oben in Gruppe 1 aufgeführten Bedeutungen.

### Gruppe 37

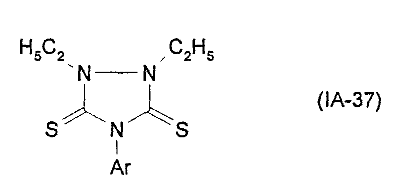

Ar hat hierbei beispielhaft die oben in Gruppe 1 aufgeführten Bedeutungen.

### Gruppe 38

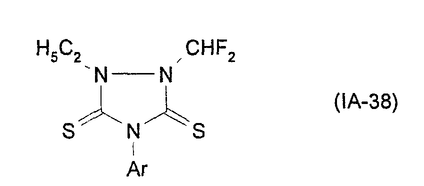

Ar hat hierbei beispielhaft die oben in Gruppe 1 aufgeführten Bedeutungen.

### Gruppe 39

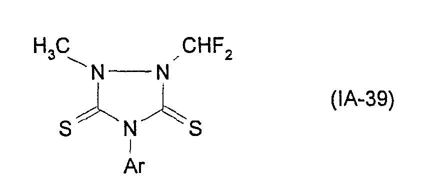

Ar hat hierbei beispielhaft die oben in Gruppe 1 aufgeführten Bedeutungen.

### Gruppe 40

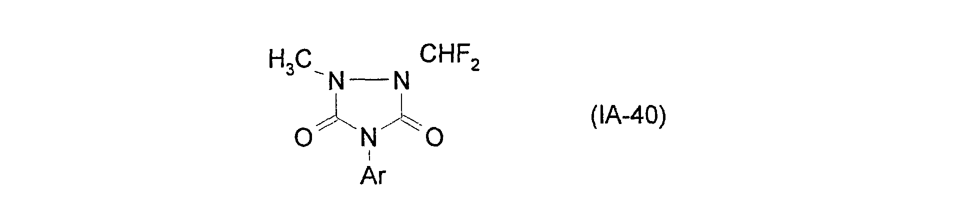

Ar hat hierbei beispielhaft die oben in Gruppe 1 aufgeführten Bedeutungen.

### Gruppe 41

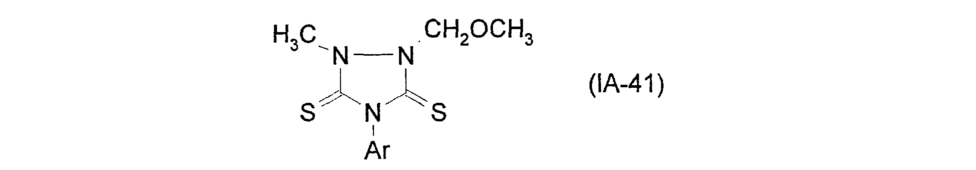

Ar hat hierbei beispielhaft die oben in Gruppe 1 aufgeführten Bedeutungen.

### Gruppe 42

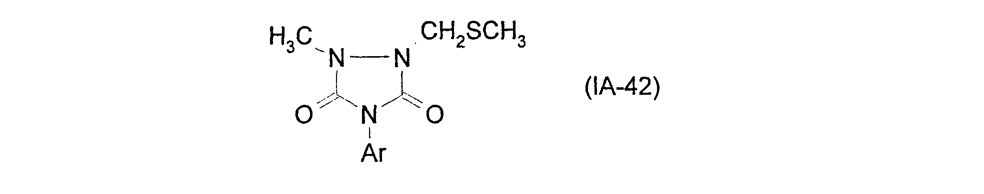

Ar hat hierbei beispielhaft die oben in Gruppe 1 aufgeführten Bedeutungen.

### Gruppe 43

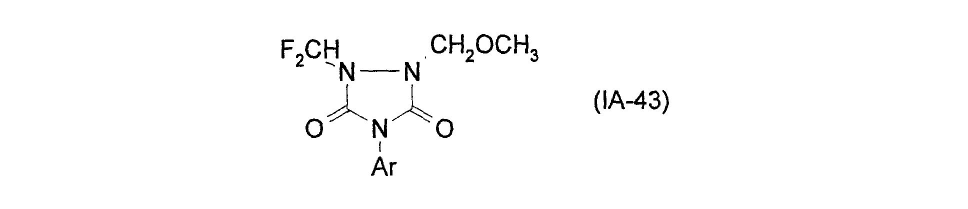

Ar hat hierbei beispielhaft die oben in Gruppe 1 aufgeführten Bedeutungen.

### Gruppe 44

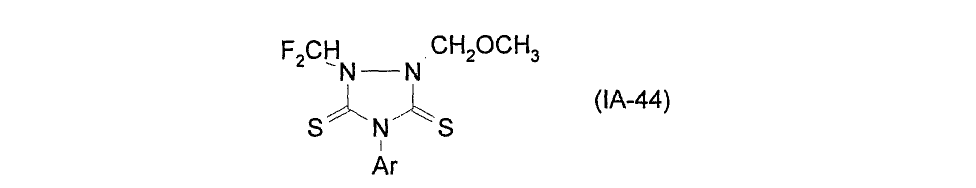

Ar hat hierbei beispielhaft die oben in Gruppe 1 aufgeführten Bedeutungen.

### Gruppe 45

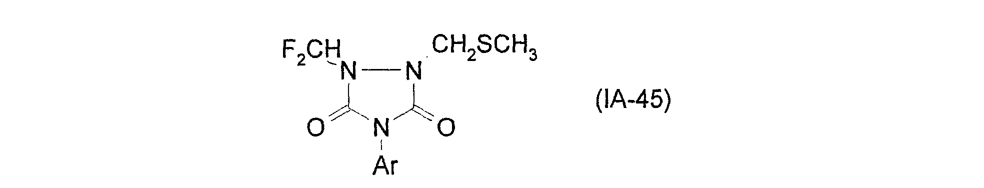

Ar hat hierbei beispielhaft die oben in Gruppe 1 aufgeführten Bedeutungen.

### Gruppe 46

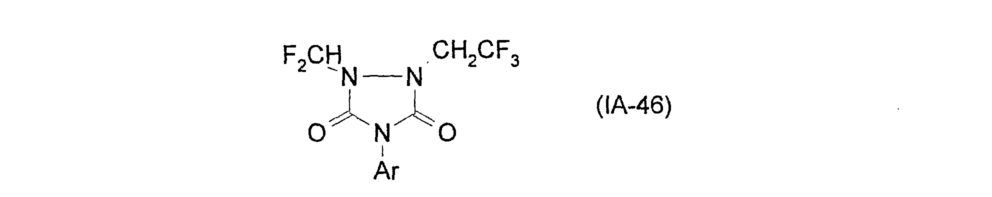

Ar hat hierbei beispielhaft die oben in Gruppe 1 aufgeführten Bedeutungen.

### Gruppe 47

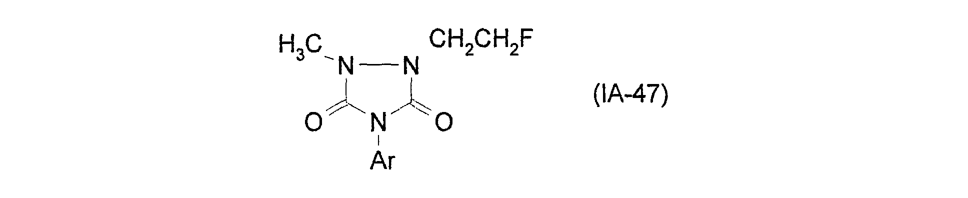

Ar hat hierbei beispielhaft die oben in Gruppe 1 aufgeführten Bedeutungen.

### Gruppe 48

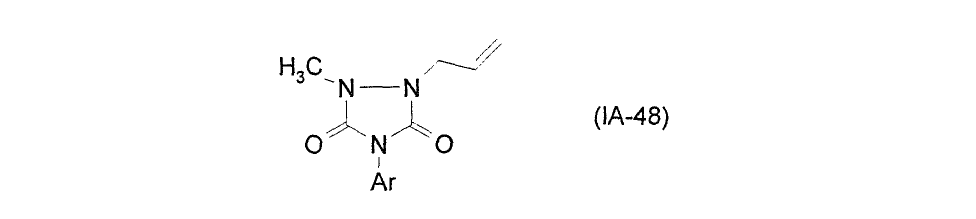

Ar hat hierbei beispielhaft die oben in Gruppe 1 aufgeführten Bedeutungen.

### Gruppe 49

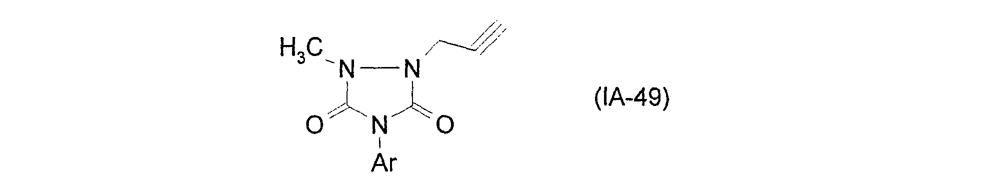

Ar hat hierbei beispielhaft die oben in Gruppe 1 aufgeführten Bedeutungen.

### Gruppe 50

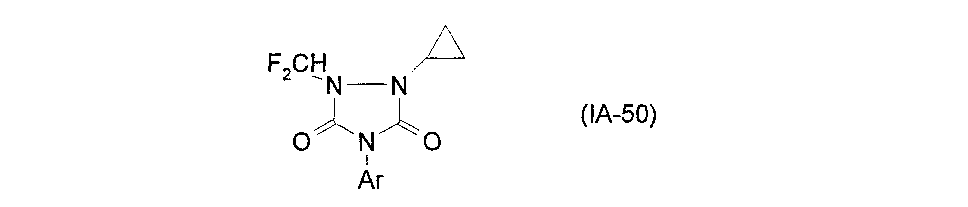

Ar hat hierbei beispielhaft die oben in Gruppe 1 aufgeführten Bedeutungen.

### Gruppe 51

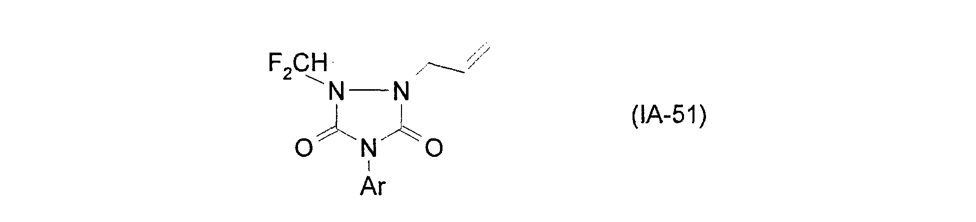

Ar hat hierbei beispielhaft die oben in Gruppe 1 aufgeführten Bedeutungen.

### Gruppe 52

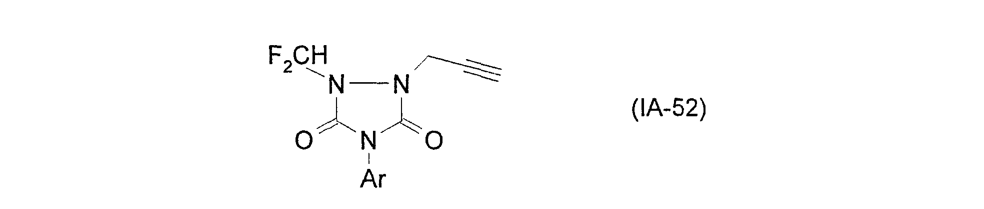

Ar hat hierbei beispielhaft die oben in Gruppe 1 aufgeführten Bedeutungen.

### Gruppe 53

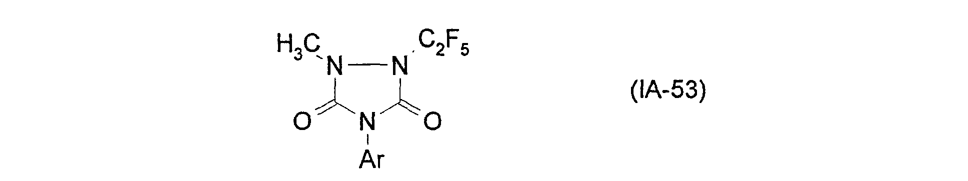

Ar hat hierbei beispielhaft die oben in Gruppe 1 aufgeführten Bedeutungen.

### Gruppe 54

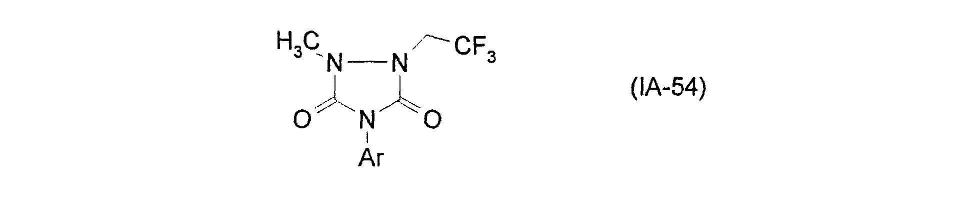

Ar hat hierbei beispielhaft die oben in Gruppe 1 aufgeführfen Bedeutungen.

### Gruppe 55

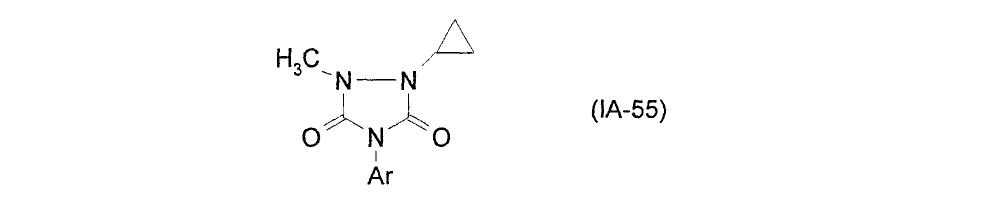

Ar hat hierbei beispielhaft die oben in Gruppe 1 aufgeführten Bedeutungen.

### Gruppe 56

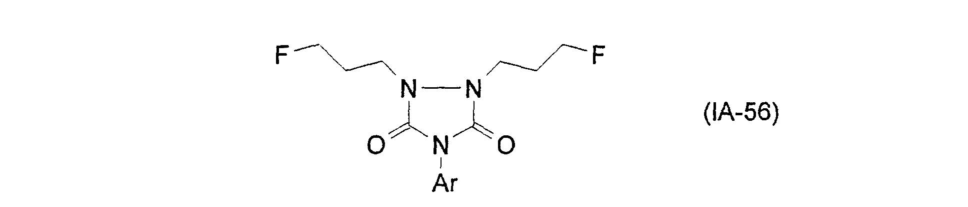

Ar hat hierbei beispielhaft die oben in Gruppe 1 aufgeführten Bedeutungen.

### Gruppe 57

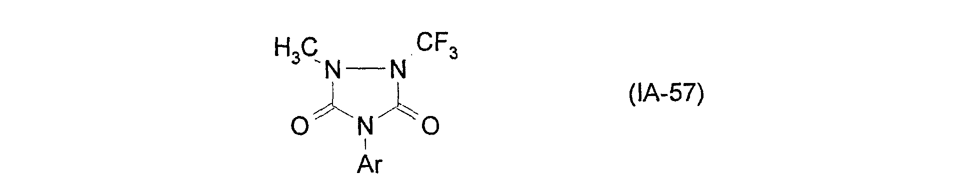

Ar hat hierbei beispielhaft die oben in Gruppe 1 aufgeführten Bedeutungen.

### Gruppe 58

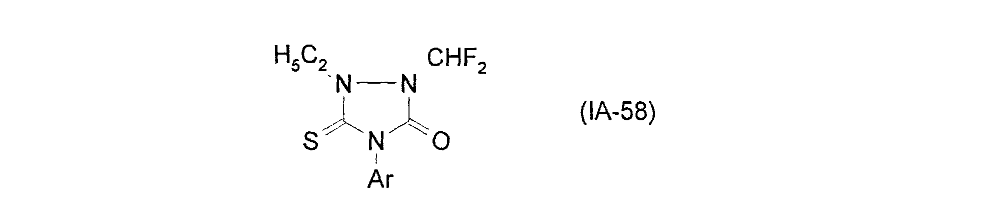

Ar hat hierbei beispielhaft die oben in Gruppe 1 aufgeführten Bedeutungen.

### Gruppe 59

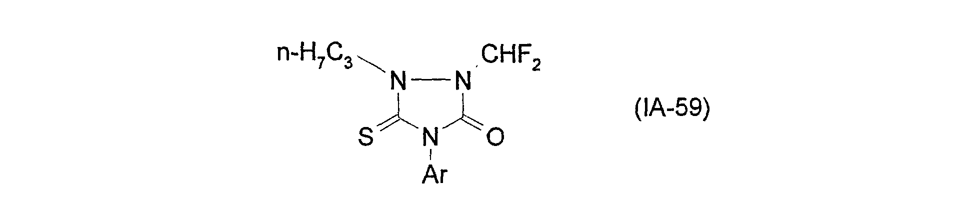

Ar hat hierbei beispielhaft die oben in Gruppe 1 aufgeführten Bedeutungen.

### Gruppe 60

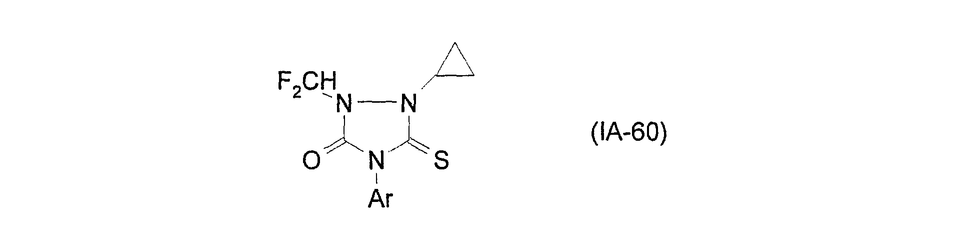

Ar hat hierbei beispielhaft die oben in Gruppe 1 aufgeführten Bedeutungen.

### Gruppe 61

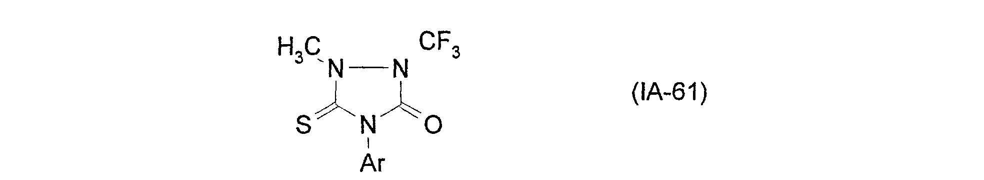

Ar hat hierbei beispielhaft die oben in Gruppe 1 aufgeführten Bedeutungen.

### Gruppe 62

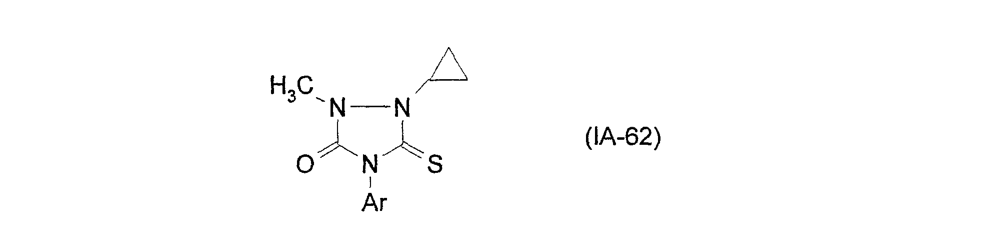

Ar hat hierbei beispielhaft die oben in Gruppe 1 aufgeführten Bedeutungen.

### Gruppe 63

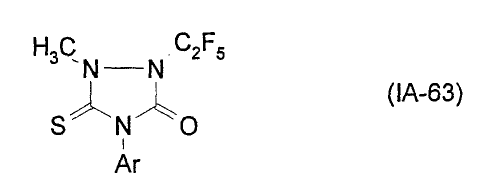

Ar hat hierbei beispielhaft die oben in Gruppe 1 aufgeführten Bedeutungen.

### Gruppe 64

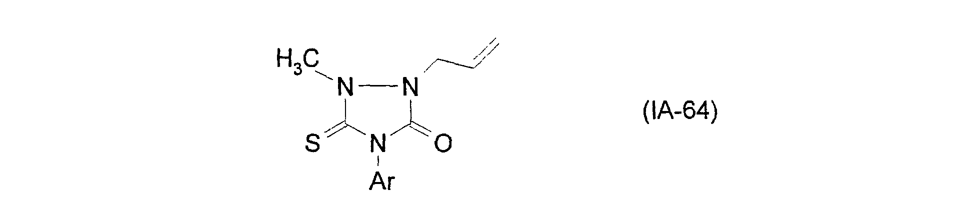

Ar hat hierbei beispielhaft die oben in Gruppe 1 aufgeführten Bedeutungen.

### Gruppe 65

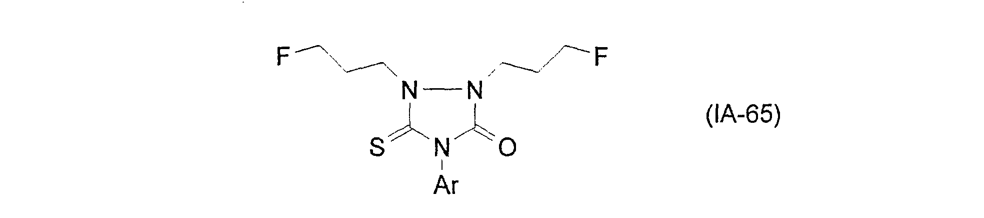

Ar hat hierbei beispielhaft die oben in Gruppe 1 aufgeführten Bedeutungen.

### Gruppe 66

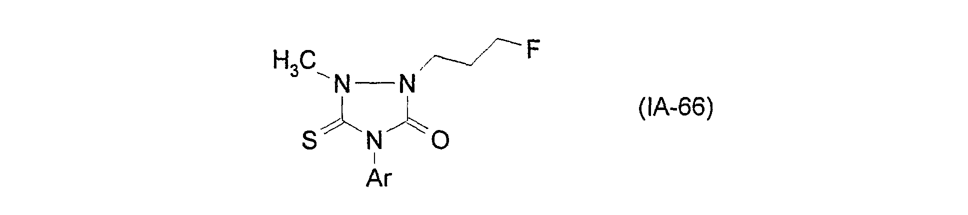

Ar hat hierbei beispielhaft die oben in Gruppe 1 aufgeführten Bedeutungen.

### Gruppe 67

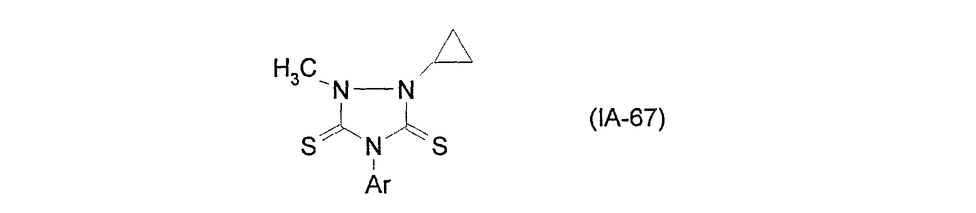

Ar hat hierbei beispielhaft die oben in Gruppe 1 aufgeführten Bedeutungen.

### Gruppe 68

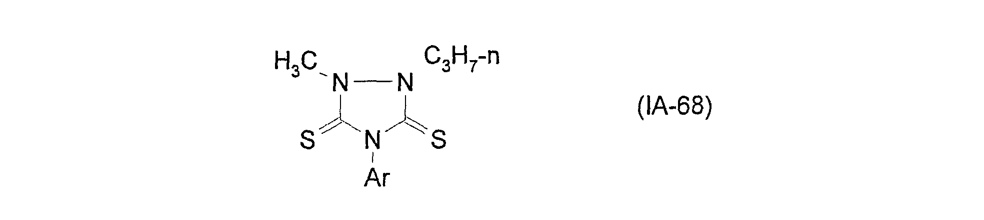

Ar hat hierbei beispielhaft die oben in Gruppe 1 aufgeführten Bedeutungen.

### Gruppe 69

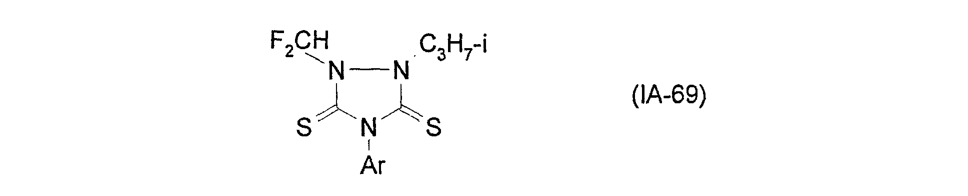

Ar hat hierbei beispielhaft die oben in Gruppe 1 aufgeführten Bedeutungen.

### Gruppe 70

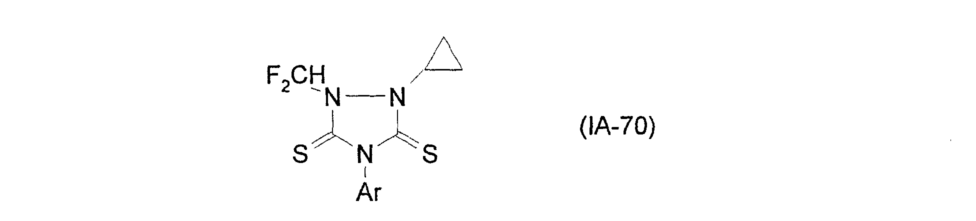

Ar hat hierbei beispielhaft die oben in Gruppe 1 aufgeführten Bedeutungen.

### Gruppe 71

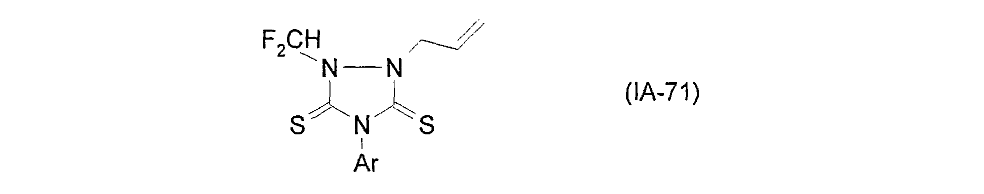

Ar hat hierbei beispielhaft die oben in Gruppe 1 aufgeführten Bedeutungen.

### Gruppe 72

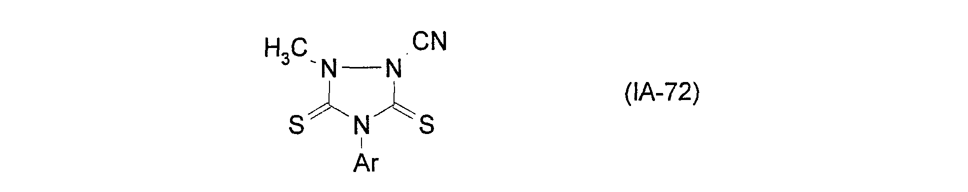

Ar hat hierbei beispielhaft die oben in Gruppe 1 aufgeführten Bedeutungen.

### Gruppe 73

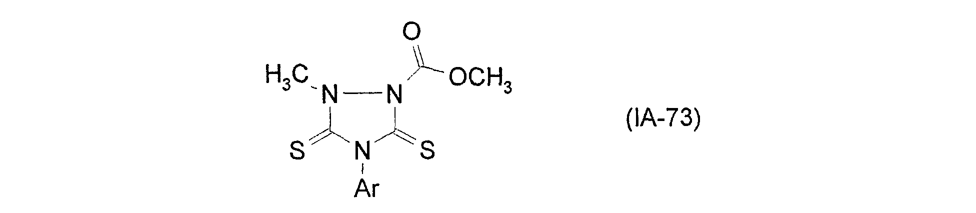

Ar hat hierbei beispielhaft die oben in Gruppe 1 aufgeführten Bedeutungen.

### Gruppe 74

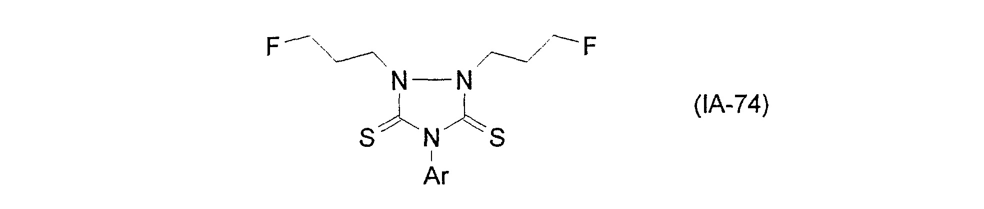

Ar hat hierbei beispielhaft die oben in Gruppe 1 aufgeführten Bedeutungen.

### Gruppe 75

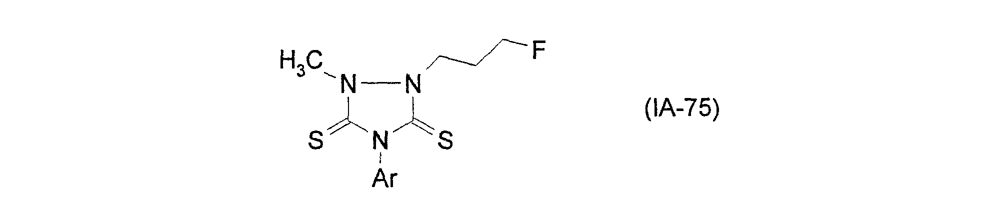

Ar hat hierbei beispielhaft die oben in Gruppe 1 aufgeführten Bedeutungen.

Die beim erfindungsgemäßen Verfahren (a) zur Herstellung der Verbindungen der allgemeinen Formel (I) als Ausgangsstoffe zu verwendenden (Thio)Semicarbazid-Derivate sind durch die Formel (II) allgemein definiert. In der Formel (II) haben Q¹, Q², R¹, R² und Ar vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q¹, Q², R¹, R² und Ar angegeben wurden; R steht vorzugsweise für Alkyl mit 1 bis 4 Kohlenstoffatomen, insbesondere für Methyl oder Ethyl.

Die Ausgangsstoffe der Formel (II) sind bekannt und/oder können nach bekannten Verfahren hergestellt werden (vgl. Synthesis 1982, 159-160; DE 1200824, DE 2952685 und DE 3026739).

Man erhält die (Thio)Semicarbazid-Derivate der Formel (II), wenn man
(α) Aryliso(thio)cyanate der allgemeinen Formel (IV)

Ar-N=C=Q¹ (IV)

in welcher
Ar und Q¹ die oben angegebenen Bedeutungen haben,
mit Carbazaten der allgemeinen Formel (XI)

RO-CQ²-N(R²)-NH-R¹ (XI)

in welcher
- Q², R¹ und R²: die oben angegebenen Bedeutungen haben und
- R: für Alkyl, vorzugsweise für C₁-C₆-Alkyl, insbesondere bevorzugt für Methyl oder Ethyl steht,
gegebenenfalls in Gegenwart eines Verdünnungsmittels, wie z.B. Toluol, bei Temperaturen zwischen 0°C und 150°C umsetzt (vgl. die Herstellungsbeispiele),
oder wenn man
(β) Arylamine der allgemeinen Formel (XII)

Ar-NH₂ (XII)

in welcher
- Ar: die oben angegebene Bedeutung hat,
mit (Thio)Carbonyl-diimidazol der Formel (XIII)

Im-CQ¹-Im (XIII)

in welcher
- Q¹: die oben angegebene Bedeutung hat und
- Im: für Imidazolyl steht,
und mit Carbazaten der allgemeinen Formel (XI)

RO-CQ²-N(R²)-NH-R¹ (XI)

in welcher
- Q², R¹ und R²: die oben angegebenen Bedeutungen haben und
- R: für Alkyl, vorzugsweise für C₁-C₆-Alkyl, insbesondere bevorzugt für Methyl oder Ethyl steht,
gegebenenfalls in Gegenwart eines Reaktionshilfsmittels, wie z.B. Kaliumhydroxid, und gegebenenfalls in Gegenwart eines Verdünnungsmittels, wie z.B. Methanol, Ethanol und/oder Wasser, bei Temperaturen zwischen 0°C und 100°C umsetzt.

Die als Vorprodukte benötigten Aryliso(thio)cyanate der Formel (IV) sind bekannt und/oder können nach bekannten Verfahren hergestellt werden (vgl. DE 4327743, DE 4335438 und DE 4343451).

Die weiter als Vorprodukte benötigten Carbazate der Formel (VIII) sind bekannte organische Chemikalien.

Das erfindungsgemäße Verfahren (a) wird vorzugsweise in Gegenwart eines geeigneten Reaktionshilfsmittels durchgeführt. Als solche kommen alle üblichen anorganischen oder organischen Basen infrage. Hierzu gehören beispielsweise Alkalimetall- oder Erdalkalimetall-hydride, -hydroxide, -amide, -alkoholate, -acetate, -carbonate oder -hydrogencarbonate, wie beispielsweise Lithium-, Natrium-, Kalium- oder Calcium-hydrid, Lithium-, Natrium- oder Kalium-amid, Natrium- oder Kaliummethylat, Natrium- oder Kalium-ethylat, Natrium- oder Kalium-propylat, Aluminiumisopropylat, Natrium- oder Kalium-tert-butylat, Natrium- oder Kaliumhydroxid, Ammoniumhydroxid, Natrium-, Kalium- oder Calcium-acetat, Ammoniumacetat, Natrium-, Kalium-, Rubidium-, Cäsium-, Magnesium- oder Calcium-carbonat, Ammoniumcarbonat, Natrium- oder Kalium-hydrogencarbonat, sowie basische organische Stickstoffverbindungen, wie Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N,N-Dimethylanilin, N,N-Dimethyl-benzylamin, Pyridin, 2-Methyl-, 3-Methyl- und 4-Methyl-pyridin, 2,4-Dimethyl-, 2,6-Dimethyl-, 3,4-Dimethyl- und 3,5-Dimethyl-pyridin, 5-Ethyl-2-methylpyridin, N-Methylpiperidin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (a) kommen die üblichen organischen Lösungsmittel in Betracht. Hierzu gehören insbesondere aliphatische, alicyclische oder aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan, Chloroform, Tetrachlormethan; Ether, wie Diethylether, Diisopropylether, t-Butyl-methylether, t-Pentyl-methylether, Dioxan, Tetrahydrofuran, Ethylenglykol-dimethyl- oder -diethylether, Diethylenglykol-dimethylether oder -diethylether; Ketone, wie Aceton, Butanon oder Methyl-isobutyl-keton; Nitrile, wie Acetonitril, Propionitril, Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäure-methylester, -ethylester, -n- oder -i-propylester, -n-, -i- oder -s-butylester, Sulfoxide, wie Dimethylsulfoxid; Alkohole, wie Methanol, Ethanol, n- oder i-Propanol, n-, i-, s-oder t-Butanol, Ethylenglykol-monomethylether oder -monoethylether, Diethylenglykolmonomethylether, Diethylenglykolmonoethylether; deren Gemische mit Wasser oder reines Wasser.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und +150°C, vorzugsweise bei Temperaturen zwischen 10°C und 120°C.

Das erfmdungsgemäße Verfahren (a) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (a) werden im allgemeinen die Ausgangsstoffe der Formel (II) in einem geeigneten Verdünnungsmittel vorgelegt und - gegebenenfalls nach Zugabe eines Reaktionshilfsmittels - bei der erforderlichen Temperatur bis zum Ende der Umsetzung gerührt. Die Aufarbeitung kann auf übliche Weise erfolgen (vgl. die Herstellungsbeispiele).

Die beim erfindungsgemäßen Verfahren (b) zur Herstellung der Verbindungen der allgemeinen Formel (I) als Ausgangsstoffe zu verwendenden Aryliminoheterocyclen sind durch die Formel (III) allgemein definiert. In der Formel (III) haben Q¹, Q², R¹, R² und Ar vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q¹, Q², R¹, R² und Ar angegeben wurden.

Die Ausgangsstoffe der Formel (III) sind bisher noch nicht aus der Literatur bekannt; sie sind jedoch Gegenstand einer vorgängigen, nicht vorveröffentlichten Anmeldung (vgl. DE 4424787).

Man erhält die Aryliminoheterocyclen der Formel (III), wenn man Aryl(thio)semicarbazide der allgemeinen Formel (VI) mit reaktiven Kohlensäurederivaten, wie z.B. Phosgen oder Thiophosgen, gegebenenfalls in Gegenwart von Verdünnungsmitteln, wie z.B. Toluol und/oder Dichlormethan, bei Temperaturen zwischen 0°C und 100°C umsetzt.

Es kommen hierbei vorzugsweise die gleichen Verdünnungsmittel und Reaktionshilfsmittel wie bei beim erfindungsgemäßen Verfahren (a) in Betracht.

Das erfindungsgemäße Verfahren (b) wird gegebenenfalls in Gegenwart eines Reaktionshilfsmittels durchgeführt. Es kommen hierbei die gleichen Reaktionshilfsmittel wie beim erfindungsgemäßen Verfahren (a), außerdem jedoch auch noch Alkalimetallsulfide, wie z.B. Natrium- oder Kaliumsulfid, in Betracht.

Das erfindungsgemäße Verfahren (b) wird vorzugsweise unter Verwendung eines Verdünnungsmittels durchgeführt. Es kommen hierbei die gleichen Verdünnungsmittel wie beim erfindungsgemäßen Verfahren (a) in Betracht.

Die Reaktionstemperaturen können bei der Durchführung des erfindungsgemäßen Verfahrens (b) in einem größeren Bereich variiert werden. Im allgemeinen arbeitet man bei Temperaturen zwischen 0°C und +250°C, vorzugsweise bei Temperaturen zwischen 20°C und 150°C.

Das erfindungsgemäße Verfahren (b) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (b) werden im allgemeinen die Ausgangsstoffe der Formel (III) in einem geeigneten Verdünnungsmittel vorgelegt und bei der erforderlichen Temperatur bis zum Ende der Umsetzung gerührt. Die Aufarbeitung kann auf übliche Weise erfolgen.

Die erfindungsgemäßen Wirkstoffe können als Defoliants, Desiccants, Krautabtötungsmittel und insbesondere als Unkrautvemichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindemia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.
Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolin-säure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylhamstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfüron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

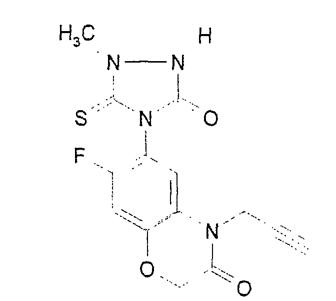

Eine Mischung aus 7,6 g (23 mMol) 2-(7-Fluor-3,4-dihydro-3-oxo-4-propargyl-(2H)-1,4-benzoxazin-6-yl-imino)-3-methyl-3,4-dihydro-5-oxo-(4H)-1,3,4-thiadiazol und 20 ml Dimethylsulfoxid wird 2 Stunden auf 50°C, weitere 2 Stunden bei 70°C und weitere 2 Stunden bei 80°C erwärmt. Dann wird unter vermindertem Druck eingeengt, der Rückstand mit Wasser verrührt und das kristallin anfallende Produkt durch Abfiltrieren isoliert.

Man erhält 6,6 g (85% der Theorie) 4-(7-Fluor-3,4-dihydro-3-oxo-4-propargyl-(2H)-1,4-benzoxazin-6-yl)-1-methyl-5-thioxo-1,2,4-triazolin-3-on vom Schmelzpunkt 190°C.

Analog zu Beispiel 1 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

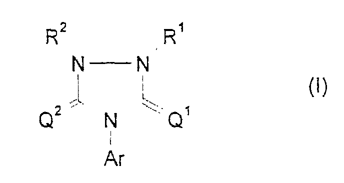

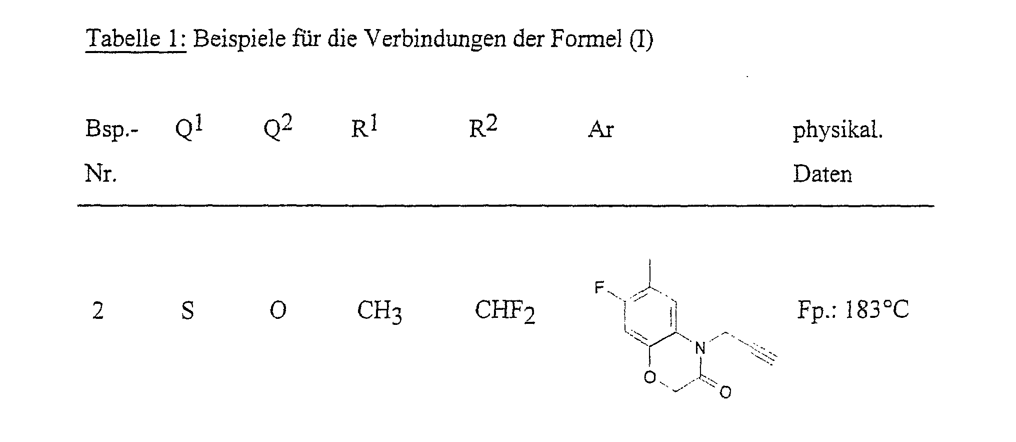

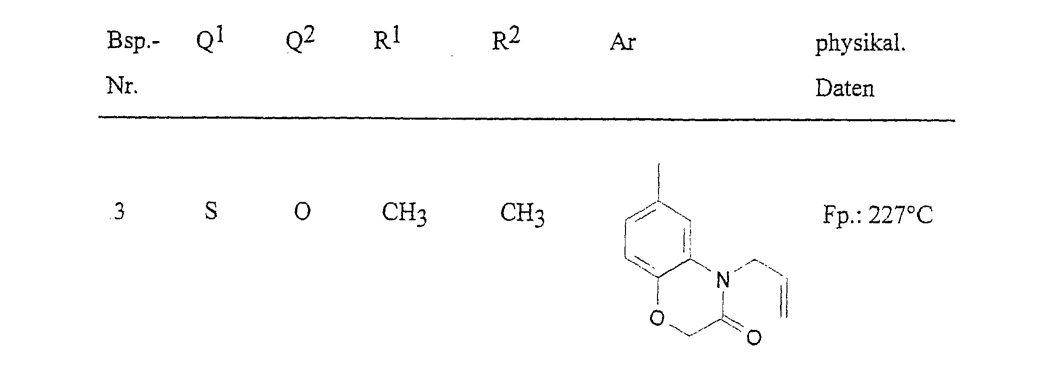

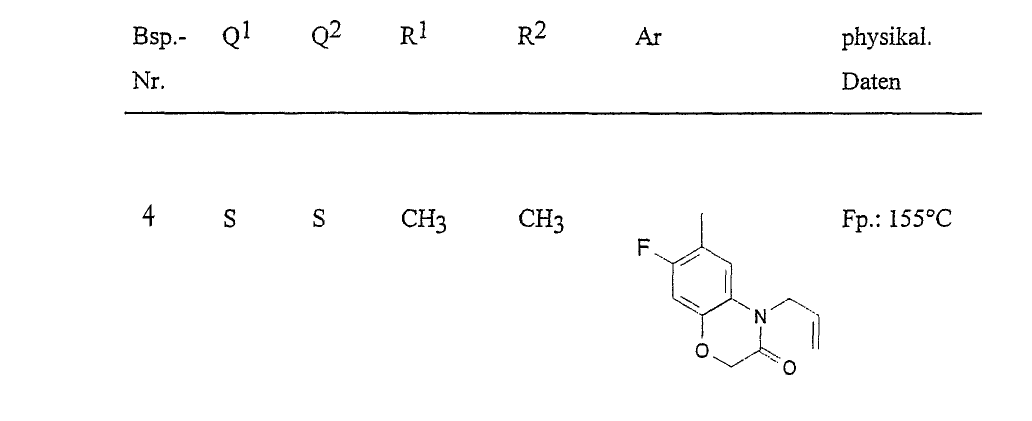

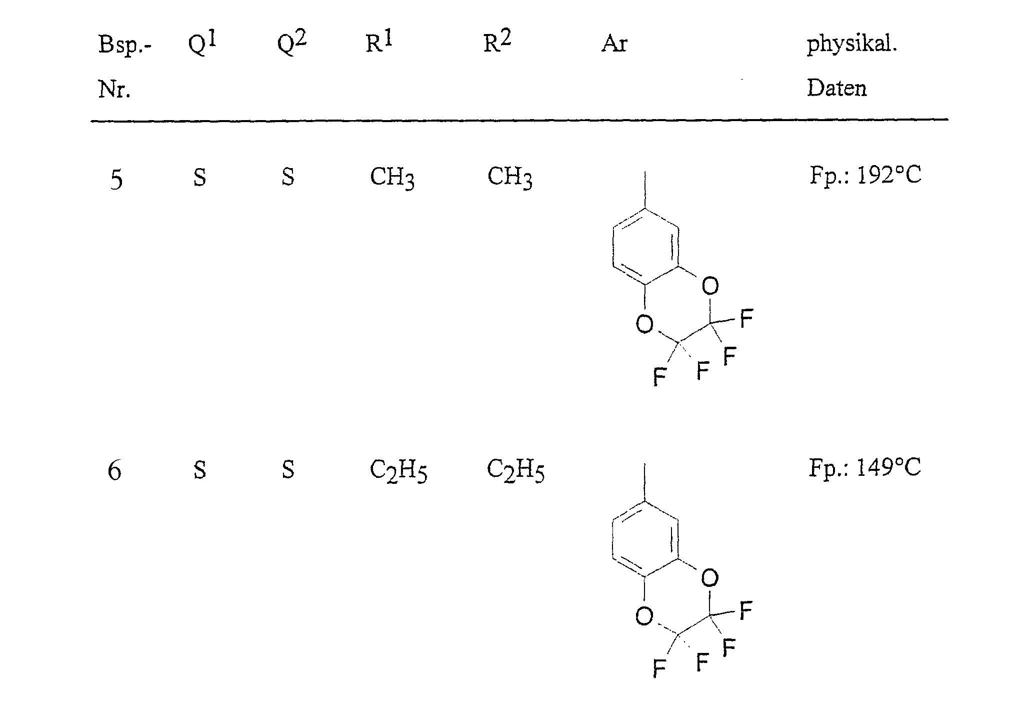

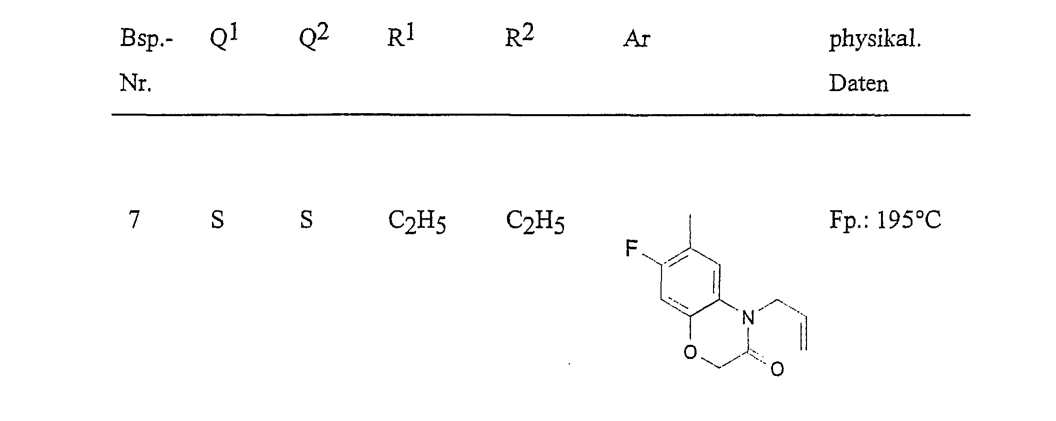

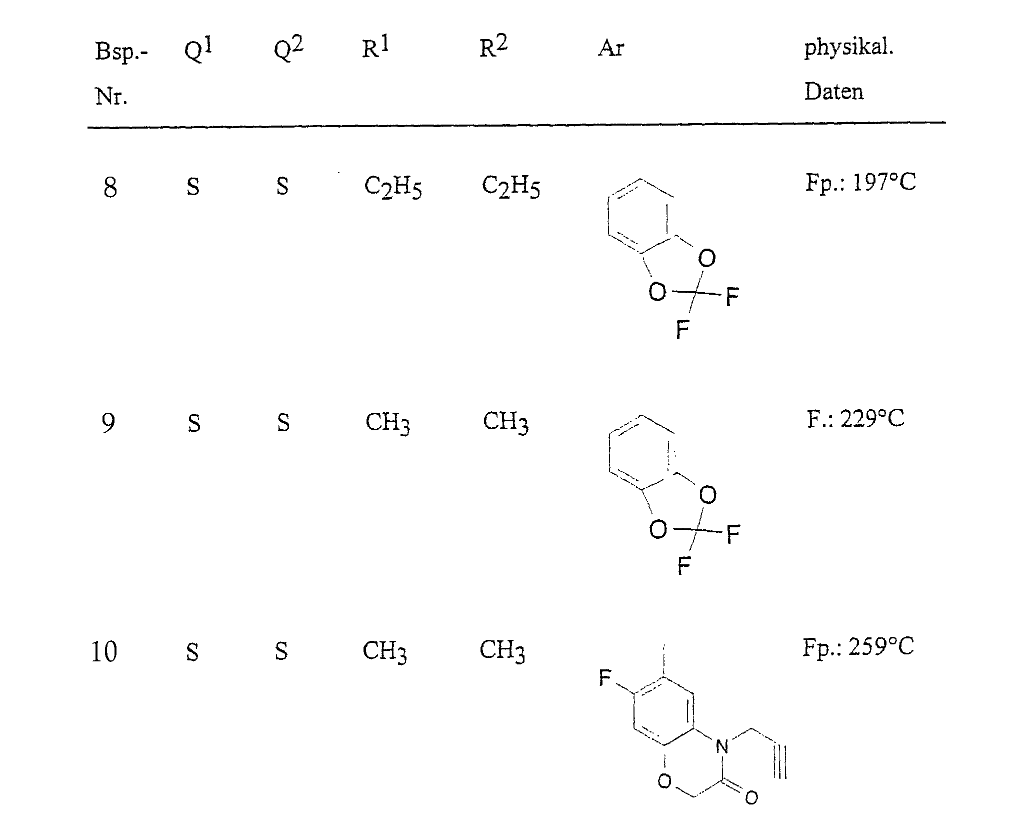

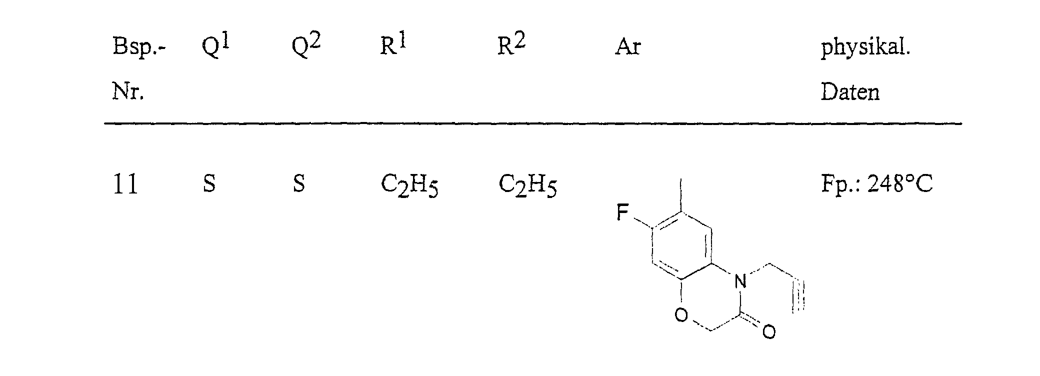

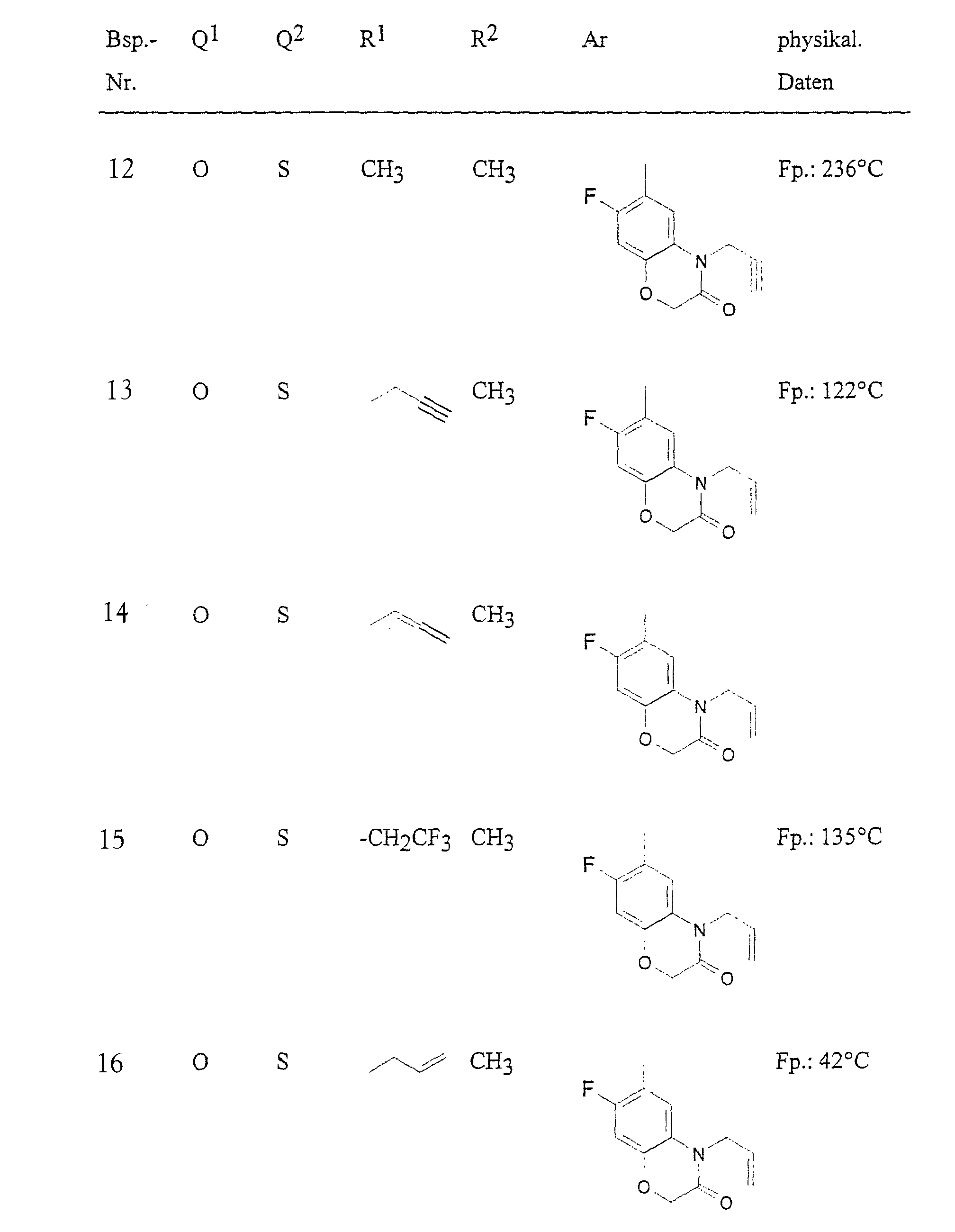

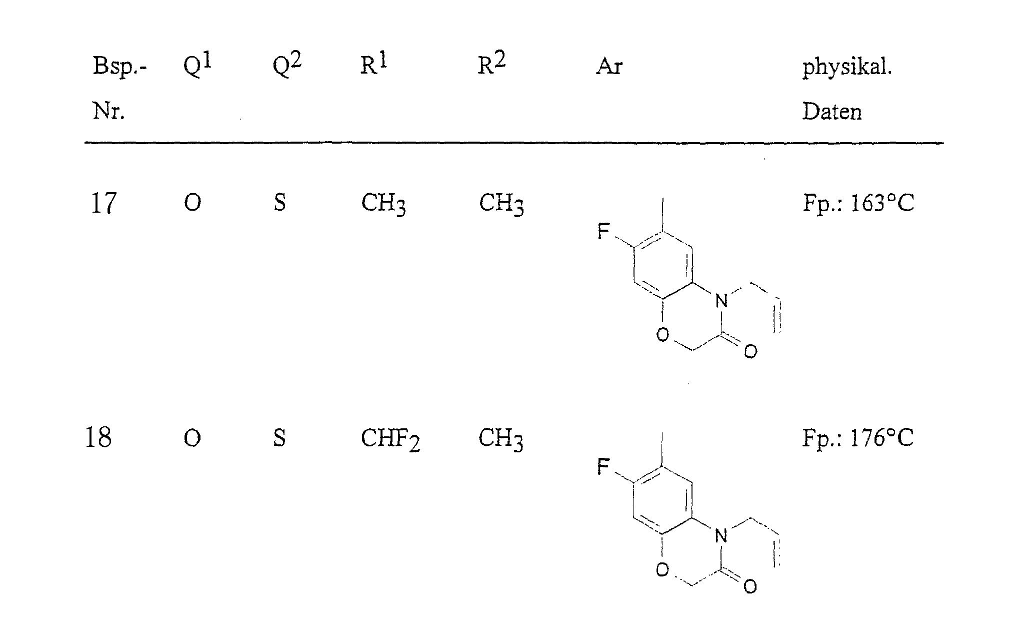

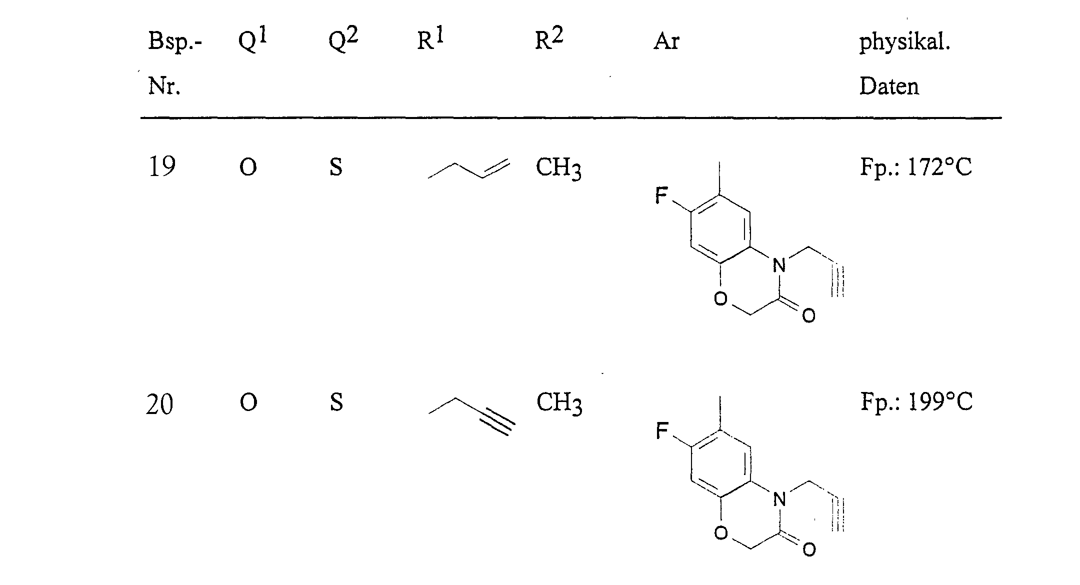

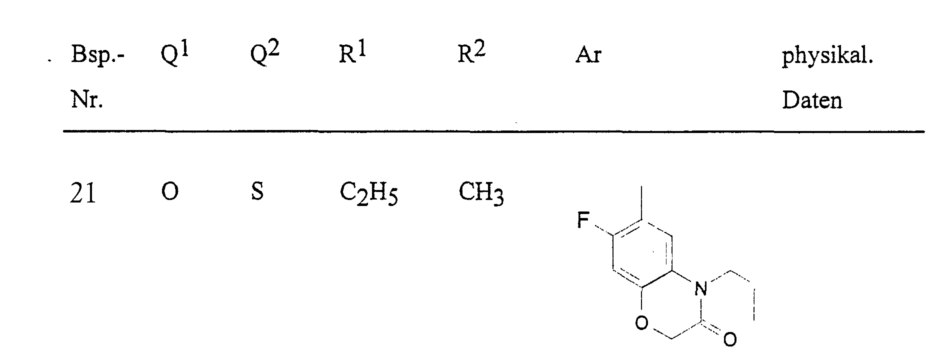

Die in Tabelle 1 als Beispiel 12 aufgeführte Verbindung kann beispielsweise wie folgt hergestellt werden:

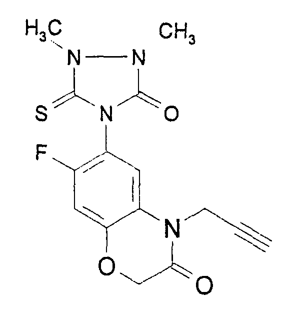

Eine Lösung von 2,1 g (6 mMol) 4-(7-Fluor-3,4-dihydro-3-oxo-4-propargyl-(2H)-1,4-benzoxazin-6-yl)-1-methyl-5-methylthio-1,2,4-triazolin-3-on in 20 ml Dimethylformamid wird 16 Stunden unter Rückfluß erhitzt. Anschließend wird im Wasserstrahlvakuum eingeengt und der Rückstand durch Säulenchromatographie gereinigt.

Man erhält 0,9 g (43% der Theorie) 4-(7-Fluor-3,4-dihydro-3-oxo-4-propargyl-(2H)-1,4-benzoxazin-6-yl)-1,2-dimethyl-5-thioxo-1,2,4-triazolin-3-on vom Schmelzpunkt 236°C.

### Ausgangsstoffe der Formel (III):

### Beispiel (III-1)

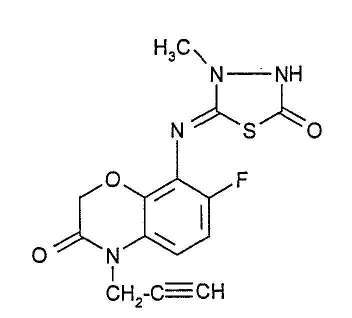

Zu einer Suspension von 3,1 g (10 mMol) 2-Methyl-4-(7-fluor-3,4-dihydro-3-oxo-4-propargyl-(2H)-1,4-benzoxazin-6-yl)-thiosemicarbazid in 50 ml Dichlormethan gibt man bei ca. 20°C 6 g (12 mMol) einer 20%igen Lösung von Phosgen in Toluol. Man erwärmt die Reaktionsmischung ca. 15 Stunden auf 40°C, entfernt das Lösungsmittel im Vakuum und nimmt den Rückstand in Wasser auf. Man neutralisiert mit Natriumbicarbonatlösung, filtriert den Feststoff, wäscht mit Wasser und trocknet im Vakuum bei 40-50°C.

Man erhält 2,8 g (84% der Theorie) 2-(7-Fluor-3,4-dihydro-3-oxo-4-propargyl-(2H)-1,4-benzoxazin-6-yl-imino)-3-methyl-3,4-dihydro-5-oxo-(4H)-1,3,4-thiadiazol.
Schmelzpunkt: 214°C.

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
- 0 % =: keine Wirkung (wie unbehandelte Kontrolle)
- 100 % =: totale Vernichtung

In diesem Test zeigen beispielsweise die Verbindungen gemäß den Herstellungsbeispielen 2 und 3 bei Aufwandmengen von 60 g/ha teilweise gute Verträglichkeit gegenüber Kulturpflanzen, wie z.B. Gerste und Mais (10-70 %) und sehr starke Wirkung gegen Unkräuter wie Alopecurus (90-100 %), Cynodon (95 -100 %), Setaria (70-100 %), Amaranthus (90-100 %), Chenopodium (100 %), Matricaria (95-100%), Polygonum (80-100 %), Portulaca (95-100 %) und Viola (90-100 %).

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.
Es bedeuten:
- 0 % =: keine Wirkung (wie unbehandelte Kontrolle)
- 100 % =: totale Vernichtung

In diesem Test zeigen beispielsweise die Verbindungen gemäß den Herstellungsbeispielen 1, 2 und 3 bei Aufwandmengen zwischen 15 und 250 g/ha starke Wirkung gegen Unkräuter wie Amaranthus (60-100 %), Chenopodium (90-100 %), Datura (80-100 %), Galium (90-100 %) und Veronica (50-100 %).

## Patentansprüche

1. Substituierte N-Aryl-Stickstoffheterocyclen der allgemeinen Formel (I) 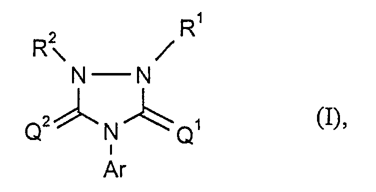 in welcher
Q¹ für Sauerstoff oder Schwefel steht,
Q² für Sauerstoff oder Schwefel steht,
R¹ für Wasserstoff, Cyano, Formyl oder für jeweils gegebenenfalls durch Fluor, Chlor, Cyano, Carboxy, C₁-C₄-Alkoxy, C₃-C₄-Alkenyloxy, C₃-C₄-Alkinyloxy, C₁-C₄-Alkylthio, C₃-C₄-Alkenylthio, C₃-C₄-Alkinylthio, C₁-C₄-Alkoxy-carbonyl, C₃-C₄-Alkenyloxy-carbonyl oder C₃-C₄-Alkinyloxy-carbonyl substituiertes C₁-C₆-Alkyl steht,
R¹ weiterhin für jeweils gegebenenfalls durch Fluor oder Chlor substituiertes C₃-C₆-Alkenyl oder C₃-C₆-Alkinyl steht,
R¹ weiterhin für jeweils gegebenenfalls durch Fluor oder Chlor substituiertes C₁-C₆-Alkyl-carbonyl, C₃-C₆-Alkenyl-carbonyl, C₃-C₆-Alkinyl-carbonyl, C₁-C₆-Alkoxy-carbonyl, C₃-C₆-Alkenyloxycarbonyl oder C₃-C₆-Alkinyloxy-carbonyl steht,
R¹ weiterhin für jeweils gegebenenfalls durch Fluor, Chlor, Brom, Cyano oder Carboxy substituiertes C₃-C₆-Cycloalkyl oder C₃-C₆-Cycloalkyl-carbonyl steht,
R² für Wasserstoff, Cyano, Formyl oder für jeweils gegebenenfalls durch Fluor, Chlor, Cyano, Carboxy, C₁-C₄-Alkoxy, C₃-C₄-Alkenyloxy, C₃-C₄-Alkinyloxy, C₁-C₄-Alkylthio, C₃-C₄-Alkenylthio, C₃-C₄-Alkinylthio, C₁-C₄-Alkoxy-carbonyl, C₃-C₄-Alkenyloxy-carbonyl oder C₃-C₄-Alkinyloxy-carbonyl substituiertes C₁-C₆-Alkyl steht,
R² weiterhin für jeweils gegebenenfalls durch Fluor oder Chlor substituiertes C₃-C₆-Alkenyl oder C₃-C₆-Alkinyl steht,
R² weiterhin für jeweils gegebenenfalls durch Fluor oder Chlor substituiertes C₁-C₆-Alkyl-carbonyl, C₃-C₆-Alkenyl-carbonyl, C₃-C₆-Alkinyl-carbonyl, C₁-C₆-Alkoxy-carbonyl, C₃-C₆-Alkenyloxycarbonyl oder C₃-C₆-Alkinyloxy-carbonyl steht,
R² weiterhin für jeweils gegebenenfalls durch Fluor, Chlor, Brom, Cyano oder Carboxy substituiertes C₃-C₆-Cycloalkyl oder C₃-C₆-Cycloalkyl-carbonyl steht, und
Ar für die nachstehend definierte substituierte, bicyclische Heteroaryl-Gruppierung steht, 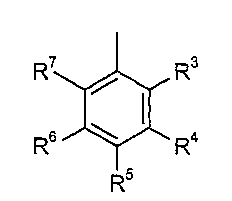
worin
R³ für Wasserstoff, Fluor, Chlor oder Brom steht,
R⁴ für Wasserstoff, Fluor, Chlor oder Brom steht,
R⁷ für Wasserstoff, Fluor oder Chlor steht,
R⁵ und R⁶ - zusammen für eine der nachstehenden Gruppierungen stehen
-Q³-C(R⁸,R⁹)-Q⁵-,
Q³-C(R⁸,R⁹)-C(R⁸,R⁹)-Q⁵-,
-Q³-CQ⁴-N(R¹⁰)-,
-Q³-C(R⁸,R⁹)-CQ⁴-N(R¹⁰)-,
-C(R⁸,R⁹)-C(R⁸,R⁹)-CQ⁴-N(R¹⁰)-,
-C(R⁸)=C(R⁸)-CQ⁴-N(R¹⁰)-,
-C(R⁸,R⁹)-CQ⁴-N(R¹⁰)-,
-N(R¹⁰)-C(R⁸,R⁹)-CQ⁴-N(R¹⁰)-,
wobei
Q³, Q⁴ und Q⁵ gleich oder verschieden sind und jeweils für Sauerstoff oder Schwefel stehen,
R⁸ und R⁹ gleich oder verschieden sind und einzeln für Wasserstoff, Fluor, Chlor, Brom oder C₁-C₄-Alkyl stehen, und
R¹⁰ für Wasserstoff, Hydroxy oder für gegebenenfalls durch Cyano, Fluor, Chlor, C₁-C₄-Alkoxy, C₁-C₄-Alkyl-carbonyl oder C₁-C₄-Alkoxycarbonyl substituiertes Alkyl, Alkylcarbonyl, Alkoxycarbonyl oder Alkylsulfonyl mit jeweils 1 bis 6 Kohlenstoffatomen in den Alkylgruppen steht,
R¹⁰ weiterhin für jeweils gegebenenfalls durch Fluor, Chlor oder Brom substituiertes Alkenyl oder Alkinyl mit jeweils 2 bis 6 Kohlenstoffatomen steht,
R¹⁰ weiterhin für jeweils gegebenenfalls durch Fluor, Chlor, Brom oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 3 Atomen in der Alkylgruppe steht,
R¹⁰ weiterhin für jeweils gegebenenfalls durch Fluor und/oder Chlor substituiertes Alkoxy oder Alkenyloxy mit jeweils bis zu 6 Kohlenstoffatomen steht,
R¹⁰ weiterhin für jeweils gegebenenfalls durch Cyano, Fluor, Chlor, C₁-C₄-Alkyl, C₁-C₄-Halogenalkyl, C₁-C₄-Alkoxy oder C₁-C₄-Halogenalkoxy substituiertes Benzyl oder Benzyloxy steht.

2. Substituierte N-Aryl-Stickstoffheterocyclen gemäß Anspruch 1, **dadurch gekennzeichnet, dass**
Q¹ für Sauerstoff oder Schwefel steht,
Q² für Sauerstoff oder Schwefel steht,
R¹ für Wasserstoff, Cyano, Formyl oder für jeweils gegebenenfalls durch Fluor, Chlor, Cyano, Carboxy, Methoxy oder Ethoxy substituiertes Methyl, Ethyl, n- oder i-Propyl, n-, i-, s- oder t-Butyl steht,
R¹ weiterhin für jeweils gegebenenfalls durch Fluor oder Chlor substituiertes Propenyl, Butenyl, Propinyl oder Butinyl steht,
R¹ weiterhin für jeweils gegebenenfalls durch Fluor oder Chlor substituiertes Acetyl, Propionyl, Methoxycarbonyl oder Ethoxycarbonyl steht,
R¹ weiterhin für gegebenenfalls durch Fluor oder Chlor substituiertes Cyclopropyl steht,
R² für Wasserstoff, Cyano, Formyl oder für jeweils gegebenenfalls durch Fluor, Chlor, Cyano, Carboxy, Methoxy oder Ethoxy substituiertes Methyl, Ethyl, n- oder i-Propyl, n-, i-, s- oder t-Butyl steht,
R² weiterhin für jeweils gegebenenfalls durch Fluor oder Chlor substituiertes Propenyl, Butenyl, Propinyl oder Butinyl steht,
R² weiterhin für jeweils gegebenenfalls durch Fluor oder Chlor substituiertes Acetyl, Propionyl, Methoxycarbonyl oder Ethoxycarbonyl steht,
R² weiterhin für gegebenenfalls durch Fluor oder Chlor substituiertes Cyclopropyl steht, und
Ar für die nachstehend definierte substituierte, bicyclische Heteroaryl-Gruppierung steht, 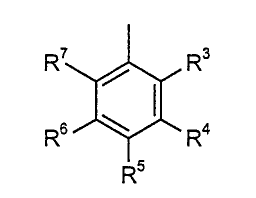
worin
R³ für Wasserstoff, Fluor oder Chlor steht,
R⁴ für Wasserstoff, Fluor oder Chlor oder steht,
R⁷ für Wasserstoff, Fluor oder Chlor steht,
R⁵ und R⁶ - zusammen für eine der nachstehenden Gruppierungen stehen
-Q³-C(R⁸,R⁹)-Q⁵-,
-Q³-C(R⁸,R⁹)-C(R⁸,R⁹)-Q⁵-,
-Q³-CQ⁴-N(R¹⁰)-,
-Q³-C(R⁸,R⁹)-CQ⁴-N(R¹⁰)-,
-C(R⁸,R⁹)-C(R⁸,R⁹)-CQ⁴-N(R¹⁰)-,
-C(R⁸)=C(R⁸)-CQ⁴-N(R¹⁰)-,
-C(R⁸,R⁹)-CQ⁴-N(R¹⁰)-,
-N(R¹⁰)-C(R⁸,R⁹)-CQ⁴-N(R¹⁰)-,
wobei
Q³, Q⁴ und Q⁵ gleich oder verschieden sind und jeweils für Sauerstoff oder Schwefel stehen,
R⁸ und R⁹ gleich oder verschieden sind und einzeln für Wasserstoff, Fluor, Chlor, Methyl oder Ethyl stehen, und
R¹⁰ für Wasserstoff, Hydroxy oder für gegebenenfalls durch Cyano, Fluor, Chlor, Methoxy, Ethoxy, Acetyl, Propionyl, Methoxycarbonyl oder Ethoxy-carbonyl substituiertes Methyl, Ethyl, n- oder i-Propyl, n-, i-,s oder t-Butyl steht,
R¹⁰ weiterhin für jeweils gegebenenfalls durch Fluor, Chlor oder Brom substituiertes Propenyl, Butenyl, Propinyl oder Butinyl steht,
R¹⁰ weiterhin für jeweils gegebenenfalls durch Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
R¹⁰ weiterhin für jeweils gegebenenfalls durch Fluor und/oder Chlor substituiertes Methoxy, Ethoxy, n- oder i-Propoxy, n-, i- oder s-Butoxy, Propenyloxy oder Butenyloxy steht,
R¹⁰ weiterhin für jeweils gegebenenfalls durch Cyano, Fluor, Chlor, Methyl, Ethyl, Trifluormethyl, Methoxy, Ethoxy, Difluormethoxy oder Trifluormethoxy substituiertes Benzyl oder Benzyloxy steht.

3. Verfahren zur Herstellung von substituierten N-Aryl-Stickstoffheterocyclen gemäß Ansruch 1 oder 2, **dadurch gekennzeichnet, dass** man
(a) (Thio)Semicarbazid-Derivate der allgemeinen Formel (II) 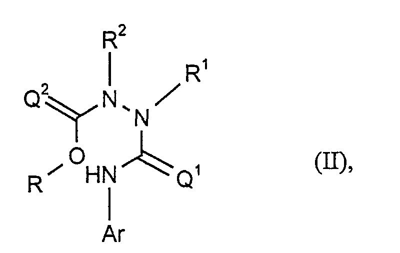 in welcher
Q¹, Q², R¹, R² und Ar die in Anspruch 1 oder 2 angegebenen Bedeutungen haben und
R für Alkyl steht,
gegebenenfalls in Gegenwart eines Reaktionshilfsmittels und gegebenenfalls in Gegenwart eines Verdünnungsmittels cyclisierend kondensiert und gegebenenfalls im Anschluss daran im Rahmen der Substituentendefinition auf übliche Weise elektrophile oder nucleophile Substitutionsreaktionen durchführt,
oder dass man
(b) Aryliminoheterocyclen der allgemeinen Formel (III) 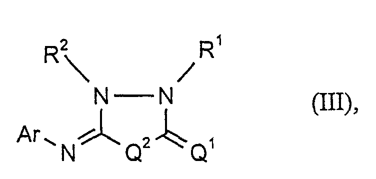 in welcher
Q¹, Q², R¹, R² und Ar die oben angegebenen Bedeutungen haben,
gegebenenfalls in Gegenwart eines Reaktionshilfsmittels und gegebenenfalls in Gegenwart eines Verdünnungsmittels thermisch ("pyrolytisch") isomerisiert.

4. Herbizide Mittel, **gekennzeichnet durch** einen Gehalt an mindestens einem N-Aryl-Stickstoffheterocyclus gemäß Anspruch 1 oder 2.

5. Verfahren zur Bekämpfung von unerwünschten Pflanzen, **dadurch gekennzeichnet, dass** man N-Aryl-Stickstoffheterocyclen gemäß Anspruch 1 oder 2 auf unerwünschte Pflanzen und/oder ihren Lebensraum einwirken lässt.

6. Verwendung von N-Aryl-Stickstoffheterocyclen gemäß Anspruch 1 oder 2 zur Bekämpfung von unerwünschten Pflanzen.

7. Verfahren zur Herstellung von herbiziden Mitteln, **dadurch gekennzeichnet, dass** man N-Aryl-Stickstoffheterocyclen gemäß Anspruch 1 oder 2 mit Streckmitteln und/oder oberflächenaktiven Substanzen vermischt.

## Claims

1. Substituted N-aryl nitrogen heterocycles of the general formula (I) 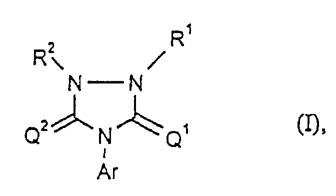 in which
Q¹ represents oxygen or sulphur,
Q² represents oxygen or sulphur,
R¹ represents hydrogen, cyano, formyl, or represents C₁-C₆-alkyl which is optionally substituted by fluorine, chlorine, cyano, carboxyl, C₁-C₄-alkoxy, C₃-C₄-alkenyloxy, C₃-C₄-alkinyloxy, C₁-C₄-alkylthio, C₃-C₄-alkenylthio, C₃-C₄-alkinylthio, C₁-C₄-alkoxy-carbonyl, C₃-C₄-alkenyloxy-carbonyl or C₃-C₄-alkinyloxy-carbonyl,
R¹ furthermore represents C₃-C₆-alkenyl or C₃-C₆-alkinyl, each of which is optionally substituted by fluorine or chlorine,
R¹ furthermore represents C₁-C₆-alkyl-carbonyl, C₃-C₆-alkenyl-carbonyl, C₃-C₆-alkinyl-carbonyl, C₁-C₆-alkoxy-carbonyl, C₃-C₆-alkenyloxy-carbonyl or C₃-C₆-alkinyloxy-carbonyl, each of which is optionally substituted by fluorine or chlorine,
R¹ furthermore represents C₃-C₆-cycloalkyl or C₃-C₆-cycloalkyl-carbonyl, each of which is optionally substituted by fluorine, chlorine, bromine, cyano or carboxyl,
R² represents hydrogen, cyano, formyl or represents C₁-C₆-alkyl which is optionally substituted by fluorine, chlorine, cyano, carboxyl, C₁-C₄-alkoxy, C₃-C₄-alkenyloxy, C₃-C₄-alkinyloxy, C₁-C₄-alkylthio, C₃-C₄-alkenylthio, C₃-C₄-alkinylthio, C₁-C₄-alkoxy-carbonyl, C₃-C₄-alkenyloxy-carbonyl or C₃-C₄-alkinyloxy-carbonyl,
R² furthermore represents C₃-C₆-alkenyl or C₃-C₆-alkinyl, each of which is optionally substituted by fluorine or chlorine,
R² furthermore represents C₁-C₆-alkyl-carbonyl, C₃-C₆-alkenyl-carbonyl, C₃-C₆-alkinyl-carbonyl, C₁-C₆-alkoxy-carbonyl, C₃-C₆-alkenyloxy-carbonyl or C₃-C₆-alkinyloxy-carbonyl, each of which is optionally substituted by fluorine or chlorine,
R² furthermore represents C₃-C₆-cycloalkyl or C₃-C₆-cycloalkyl-carbonyl, each of which is optionally substituted by fluorine, chlorine, bromine, cyano or carboxyl, and
Ar represents the substituted, bicyclic heteroaryl group defined hereinbelow, 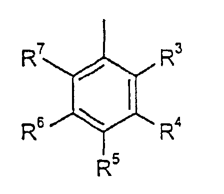
in which
R³ represents hydrogen, fluorine, chlorine or bromine,
R⁴ represents hydrogen, fluorine, chlorine or bromine,
R⁷ represents hydrogen, fluorine or chlorine,
R⁵ and R⁶ together represent one of the groups which follow
-Q³-C(R⁸,R⁹)-Q⁵-,
Q³-C(R⁸,R⁹)-C(R⁸,R⁹)-Q⁵-,
-Q³-CQ⁴-N(R¹⁰)-,
-Q³-C(R⁸,R⁹)-CQ⁴-N(R¹⁰)-,
-C(R⁸,R⁹)-C(R⁸,R⁹)-CQ⁴-N(R¹⁰)-,
-C(R⁸)=C(R⁸)-CQ⁴-N(R¹⁰)-,
-C(R⁸,R⁹)-CQ⁴-N(R¹⁰)-,
-N(R¹⁰)-C(R⁸,R⁹)-CQ⁴-N(R¹⁰)-,
where
Q³, Q⁴ and Q⁵ are identical or different and represent in each case oxygen or sulphur,
R⁸ and R⁹ are identical or different and individually represent hydrogen, fluorine, chlorine, bromine or C₁-C₄-alkyl, and
R¹⁰ represents hydrogen, hydroxyl, or represents alkyl, alkylcarbonyl, alkoxycarbonyl or alkylsulphonyl, each of which has 1 to 6 carbon atoms in the alkyl groups and is optionally substituted by cyano, fluorine, chlorine, C₁-C₄-alkoxy, C₁-C₄-alkyl-carbonyl or C₁-C₄-alkoxy-carbonyl,
R¹⁰ furthermore represents alkenyl or alkinyl, each of which has 2 to 6 carbon atoms and each of which is optionally substituted by fluorine, chlorine or bromine,
R¹⁰ furthermore represents cycloalkyl or cycloalkylalkyl, each of which has 3 to 6 carbon atoms in the cycloalkyl groups and, if appropriate, 1 to 3 atoms in the alkyl group and each of which is optionally substituted by fluorine, chlorine, bromine or C₁-C₄-alkyl,
R¹⁰ furthermore represents alkoxy or alkenyloxy, each of which has up to 6 carbon atoms and each of which is optionally substituted by fluorine and/or chlorine,
R¹⁰ furthermore represents benzyl or benzyloxy, each of which is optionally substituted by cyano, fluorine, chlorine, C₁-C₄-alkyl, C₁-C₄-halogenoalkyl, C₁-C₄-alkoxy or C₁-C₄-halogenoalkoxy.

2. Substituted N-aryl nitrogen heterocycles according to Claim 1, **characterized in that**
Q¹ represents oxygen or sulphur,
Q² represents oxygen or sulphur,
R¹ represents hydrogen, cyano, formyl, or represents methyl, ethyl, n- or i-propyl, n-, i-, s- or t-butyl, each of which is optionally substituted by fluorine, chlorine, cyano, carboxyl, methoxy or ethoxy,
R¹ furthermore represents propenyl, butenyl, propinyl or butinyl, each of which is optionally substituted by fluorine or chlorine,
R¹ furthermore represents acetyl, propionyl, methoxycarbonyl or ethoxycarbonyl, each of which is optionally substituted by fluorine or chlorine,
R¹ furthermore represents cyclopropyl which is optionally substituted by fluorine or chlorine,
R² represents hydrogen, cyano, formyl, or represents methyl, ethyl, n- or i-propyl, n-, i-, s- or t-butyl, each of which is optionally substituted by fluorine, chlorine, cyano, carboxyl, methoxy or ethoxy,
R² furthermore represents propenyl, butenyl, propinyl or butinyl, each of which is optionally substituted by fluorine or chlorine,
R² furthermore represents acetyl, propionyl, methoxycarbonyl or ethoxycarbonyl, each of which is optionally substituted by fluorine or chlorine,
R² furthermore represents cyclopropyl which is optionally substituted by fluorine or chlorine, and
Ar represents the substituted, bicyclic heteroaryl group defined hereinbelow, 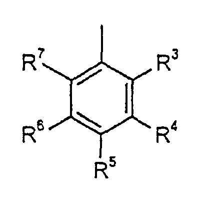
in which
R³ represents hydrogen, fluorine or chlorine,
R⁴ represents hydrogen, fluorine or chlorine,
R⁷ represents hydrogen, fluorine or chlorine,
R⁵ and R⁶ together represent one of the groups which follow
-Q³-C(R⁸,R⁹)-Q⁵-,
-Q³-C(R⁸,R⁹)-C(R⁸,R⁹)-Q⁵-,
-Q³-CQ⁴-N(R¹⁰)-,
-Q³-C(R⁸,R⁹)-CQ⁴-N(R¹⁰)-,
-C(R⁸,R⁹)-C(R⁸,R⁹)-CQ⁴-N(R¹⁰)-,
-C(R⁸)=C(R⁸)-CQ⁴-N(R¹⁰)-,
-C(R⁸,R⁹)-CQ⁴-N(R¹⁰)-,
-N(R¹⁰)-C(R⁸,R⁹)-CQ⁴-N(R¹⁰)-,
where
Q³, Q⁴ and Q⁵ are identical or different and represent in each case oxygen or sulphur,
R⁸ and R⁹ are identical or different and individually represent hydrogen, fluorine, chlorine, methyl or ethyl, and
R¹⁰ represents hydrogen, hydroxyl, or represents methyl, ethyl, n- or i-propyl, n-, i-, s- or t-butyl, each of which is optionally substituted by cyano, fluorine, chlorine, methoxy, ethoxy, acetyl, propionyl, methoxycarbonyl or ethoxy-carbonyl,
R¹⁰ furthermore represents propenyl, butenyl, propinyl or butinyl, each of which is optionally substituted by fluorine, chlorine or bromine,
R¹⁰ furthermore represents cyclopropyl, cyclobutyl, cyclopentyl, cyclohexyl, cyclopropylmethyl, cyclobutylmethyl, cyclopentylmethyl or cyclohexylmethyl, each of which is optionally substituted by fluorine, chlorine, bromine, methyl or ethyl,
R¹⁰ furthermore represents methoxy, ethoxy, n- or i-propoxy, n-, i- or s-butoxy, propenyloxy or butenyloxy, each of which is optionally substituted by fluorine and/or chlorine,
R¹⁰ furthermore represents benzyl or benzyloxy, each of which is optionally substituted by cyano, fluorine, chlorine, methyl, ethyl, trifluoromethyl, methoxy, ethoxy, difluoromethoxy or trifluoromethoxy.

3. Process for the preparation of substituted N-aryl nitrogen heterocycles according to Claim 1 or 2, **characterized in that**
(a) (thio)semicarbazide derivatives of the general formula (II) 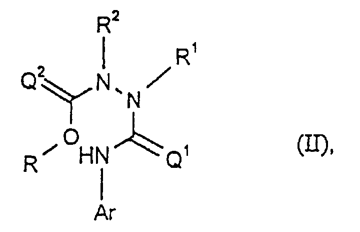 in which
Q¹, Q², R¹, R² and Ar have the meanings stated in Claim 1 or 2 and
R represents alkyl,
are subjected to a cyclizing condensation reaction, if appropriate in the presence of a reaction auxiliary and if appropriate in the presence of a diluent, and, if appropriate, electrophilic or nucleophilic substitution reactions are subsequently carried out in the customary manner within the scope of the definition of the substituents,
or **in that**
(b) arylimino heterocycles of the general formula (III) 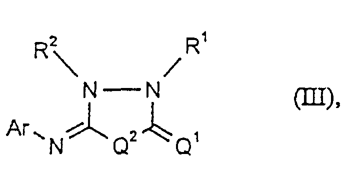 in which
Q¹, Q², R¹, R¹ and Ar have the abovementioned meanings,
are thermally ("pyrolytically") isomerized, if appropriate in the presence of a reaction auxiliary and if appropriate in the presence of a diluent.

4. Herbicidal compositions, **characterized by** a content of at least one N-aryl nitrogen heterocycle according to Claim 1 or 2.

5. Method of controlling undesired plants, **characterized in that** N-aryl nitrogen heterocycles according to Claim 1 or 2 are allowed to act on undesired plants and/or their environment.

6. Use of N-aryl nitrogen heterocycles according to Claim 1 or 2 for controlling undesirable plants.

7. Process for the preparation of herbicidal compositions, **characterized in that** N-aryl nitrogen heterocycles according to Claim 1 or 2 are mixed with extenders and/or surface-active substances.

## Revendications

1. N-aryl-hétérocycles azotés substitués de formule générale (I) 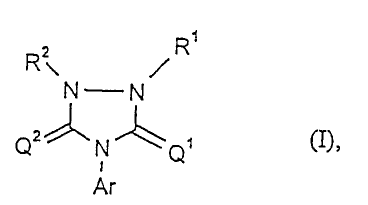 dans laquelle
Q¹ représente l'oxygène ou le soufre,
Q² représente l'oxygène ou le soufre,
R¹ est l'hydrogène, un groupe cyano, formyle, ou bien un groupe alkyle en C₁ à C₆ portant éventuellement chacun un substituant fluor, chlore, cyano, carboxy, alkoxy en C₁ à C₄, alcényloxy en C₃ ou C₄, alcynyloxy en C₃ ou C₄, alkylthio en C₁ à C₄, alcénylthio en C₃ ou C₄, alcynylthio en C₃ ou C₄, (alkoxy en C₁ à C₄)-carbonyle, (alcényloxy en C₃ à C₄)-carbonyle ou (alcynyloxy en C₃ ou C₄) -carbonyle,
R¹ représente en outre un groupe alcényle en C₃ à C₆ ou alcynyle en C₃ à C₆ éventuellement substitué chacun par du fluor ou du chlore,
R¹ représente en outre un groupe (alkyle en C₁ à C₆)-carbonyle, (alcényle en C₃ à C₆)-carbonyle, (alcynyle en C₃ à C₆)-carbonyle, (alkoxy en C₁ à C₆)-carbonyle, (alcényloxy en C₃ à C₆)-carbonyle ou (alcynyloxy en C₃ à C₆)-carbonyle dont chacun est éventuellement substitué par du fluor ou du chlore,
R¹ représente en outre un groupe cycloalkyle en C₃ à C₆ ou (cycloalkyle en C₃ à C₆)-carbonyle dont chacun est éventuellement substitué chacun par du fluor, du chlore, du brome, un radical cyano ou carboxy,
R² représente l'hydrogène, un groupe cyano, formyle ou bien un groupe alkyle en C₁ à C₆ éventuellement substitué chacun par du fluor, du chlore, un radical cyano, carboxy, alkoxy en C₁ à C₄, alcényloxy en C₃ ou C₄, alcynyloxy en C₃ ou C₄, alkylthio en C₁ à C₄, alcénylthio en C₃ ou C₄, alcynylthio en C₃ ou C₄, (alkoxy en C₁ à C₄)-carbonyle, (alcényloxy en C₃ ou C₄)-carbonyle ou (alcynyloxy en C₃ ou C₄)-carbonyle,
R² représente en outre un groupe alcynyle en C₃ à C₆ ou un groupe alcényle en C₃ à C₆ dont chacun est éventuellement substitué par du fluor ou du chlore,
R² représente en outre un groupe (alkyle en C₁ à C₆)-carbonyle, (alcényle en C₃ à C₆)-carbonyle, (alcynyle en C₃ à C₆)-carbonyle, (alkoxy en C₁ à C₆)-carbonyle, (alcényloxy en C₃ à C₆)-carbonyle ou (alcynyloxy en C₃ à C₆)-carbonyle dont chacun est éventuellement substitué par du fluor ou du chlore,
R² représente en outre un groupe cycloalkyle en C₃ à C₆ ou (cycloalkyle en C₃ à C₆)-carbonyle dont chacun est éventuellement substitué par du fluor, du chlore, du brome, un radical cyano ou carboxy, et
Ar représente le groupement hétéroaryle bicyclique substitué de formule définie ci-après 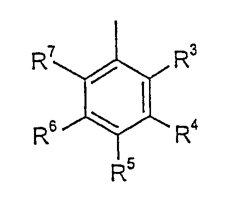
formule dans laquelle
R³ représente l'hydrogène, le fluor, le chlore ou le brome,
R⁴ représente l'hydrogène, le fluor, le chlore ou le brome,
R⁷ représente l'hydrogène, le fluor ou le chlore,
R⁵ et R⁶ forment ensemble l'un des groupements ci-après
-Q³-C(R⁸,R⁹)-Q⁵-,
Q³-C(R⁸,R⁹)-C(R⁸,R⁹)-Q⁵-,
-Q³-CQ⁴-N(R¹⁰)-,
-Q³-C(R⁸,R⁹)-CQ⁴-N(R¹⁰)-,
-C(R⁸,R⁹)-C(R⁸,R⁹)-CQ⁴-N(R¹⁰)-,
-C(R⁸)=C(R⁸)-CQ⁴-N(R¹⁰)-,
-C(R⁸,R⁹)-CQ⁴-N(R¹⁰)-,
-N(R¹⁰)-C(R⁸,R⁹)-CQ⁴-N(R¹⁰)-,
où
Q³, Q⁴ et Q⁵ sont identiques ou différents et représentent chacun l'oxygène ou le soufre,
R⁸ et R⁹ sont identiques ou différents et représentent individuellement l'hydrogène, le fluor, le chlore, le brome ou un groupe alkyle en C₁ à C₄, et
R¹⁰ représente l'hydrogène, un groupe hydroxy ou un groupe alkyle éventuellement substitué par un radical cyano, fluoro, chloro, alkoxy en C₁ à C₄, (alkyle en C₁ à C₄) -carbonyle ou (alkoxy en C₁ à C₄)-carbonyle, un groupe alkylcarbonyle, alkoxycarbonyle ou alkylsulfonyle, avec, dans chaque cas, 1 à 6 atomes de carbone dans les groupes alkyle,
R¹⁰ représente en outre un groupe alcényle ou un groupe alcynyle dont chacun a 2 à 6 atomes de carbone et chacun est éventuellement substitué par du fluor, du chlore ou du brome,
R¹⁰ représente en outre un groupe cycloalkyle ou un groupe cycloalkylalkyle ayant chacun 3 à 6 atomes de carbone dans les groupes cycloalkyle et, le cas échéant, 1 à 3 atomes de carbone dans le groupe alkyle et dont chacun est éventuellement substitué par du fluor, du chlore, du brome ou un radical alkyle en C₁ à C₄,
R¹⁰ représente en outre un groupe alkoxy ou un groupe alcényloxy ayant chacun jusqu'à 6 atomes de carbone et dont chacun est éventuellement substitué par du fluor et/ou du chlore,
R¹⁰ représente en outre un groupe benzyle ou un groupe benzyloxy dont chacun est éventuellement substitué par un radical cyano, du fluor, du chlore, un radical alkyle en C₁ à C₄, halogénalkyle en C₁ à C₄, alkoxy en C₁ à C₄ ou halogénalkoxy en C₁ à C₄.

2. N-aryl-hétérocycles azotés substitués suivant la revendication 1, **caractérisés en ce que**
Q¹ représente l'oxygène ou le soufre,
Q² représente l'oxygène ou le soufre,
R¹ est l'hydrogène, un groupe cyano, formyle ou un groupe méthyle, éthyle, n-propyle, isopropyle, n-butyle, isobutyle, sec.-butyle ou tertio-butyle dont chacun est éventuellement substitué par du fluor, du chlore, le radical cyano, carboxy, méthoxy ou éthoxy,
R¹ représente en outre un groupe propényle, butényle, propynyle ou butynyle dont chacun est éventuellement substitué par du fluor ou du chlore,
R¹ représente en outre un groupe acétyle, propionyle, méthoxycarbonyle ou éthoxycarbonyle dont chacun est éventuellement substitué par du fluor ou du chlore,
R¹ représente en outre un groupe cyclopropyle éventuellement substitué par du fluor ou du chlore,
R² représente l'hydrogène, le groupe cyano, formyle, ou un groupe méthyle, éthyle, n-propyle, isopropyle, n-butyle, isobutyle, sec.-butyle ou tertio-butyle dont chacun est éventuellement substitué par du fluor, du chlore, un radical cyano, carboxy, méthoxy ou éthoxy,
R² représente en outre un groupe propényle, butényle, propynyle ou butynyle dont chacun est éventuellement substitué par du fluor ou du chlore,
R² représente en outre un groupe acétyle, propionyle, méthoxycarbonyle ou éthoxycarbonyle dont chacun est éventuellement substitué par du fluor ou du chlore,
R² représente en outre un groupe cyclopropyle éventuellement substitué par du fluor ou du chlore, et
Ar représente le groupement hétéroaryle biscyclique substitué défini par la formule suivante 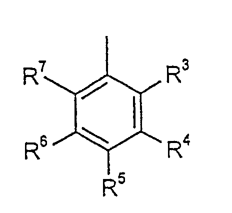
dans laquelle
R³ représente l'hdyrogène, le fluor ou le chlore,
R⁴ est l'hydrogène, le fluor ou le chlore,
R⁷ est l'hydrogène, le fluor ou le chlore,
R⁵ et R⁶ forment conjointement l'un des groupements suivants
-Q³-C(R⁸,R⁹)-Q⁵-,
-Q³-C(R⁸,R⁹)-C(R⁸,R⁹)-Q⁵-,
-Q³-CQ⁴-N(R¹⁰)-,
-Q³-C(R⁸,R⁹)-CQ⁴-N(R¹⁰)-,
-C(R⁸,R⁹)-C(R⁸,R⁹)-CQ⁴-N(R¹⁰)-,
-C(R⁸)=C(R⁸)-CQ⁴-N(R¹⁰)-,
-C(R⁸,R⁹)-CQ⁴-N(R¹⁰)-,
-N(R¹⁰)-C(R⁸,R⁹)-CQ⁴-N(R¹⁰)-,
où
Q³, Q⁴ et Q⁵ sont identiques ou différents et représentent chacun l'oxygène ou le soufre,
R⁸ et R⁹ sont identiques ou différents et représentent, individuellement, l'hydrogène, le fluor, le chlore, un groupe méthyle ou éthyle, et
R¹⁰ représente l'hydrogène, un groupe hydroxy ou un groupe méthyle, éthyle, n-propyle, isopropyle, n-butyle, isobutyle, sec.-butyle ou tertio-butyle éventuellement substitué par un radical cyano, du fluor, du chlore, un radical méthoxy, éthoxy, acétyle, propionyle, méthoxycarbonyle ou éthoxycarbonyle,
R¹⁰ représente en outre un groupe propényle, butényle, propynyle ou butynyle dont chacun est éventuellement substitué par du fluor, du chlore ou du brome,
R¹⁰ représente en outre un groupe cyclopropyle, cyclobutyle, cyclopentyle, cyclohexyle, cyclopropylméthyle, cyclobutylméthyle, cyclopentylméthyle ou cyclohexylméthyle dont chacun est éventuellement substitué par du fluor, du chlore, du brome, un radical méthyle ou éthyle,
R¹⁰ représente en outre un groupe méthoxy, éthoxy, n-propoxy, isopropoxy, n-butoxy, isobutoxy, sec.-butoxy, propényloxy ou butényloxy dont chacun est éventuellement substitué par du fluor et/ou du chlore,
R¹⁰ représente en outre un groupe benzyle ou benzyloxy dont chacun est éventuellement substitué par un radical cyano, du fluor, du chlore, un radical méthyle, éthyle, trifluorométhyle, méthoxy, éthoxy, difluorométhoxy ou trifluorométhoxy.

3. Procédé de production de N-aryl-hétérocycles azotés suivant la revendication 1 ou 2, **caractérisé en ce que** :
(a) on effectue la condensation avec cyclisation de dérivés de (thio)semicarbazide de formule générale (II) 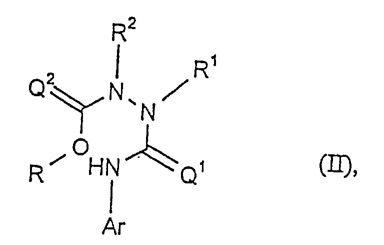 dans laquelle
Q¹, Q², R¹, R² et Ar ont les définitions indiquées dans la revendication 1 ou 2 et
R est un groupe alkyle,
le cas échéant en présence d'un auxiliaire de réaction et en présence éventuelle d'un diluant, après quoi on conduit, le cas échéant, d'une manière classique des réactions de substitution électrophile ou nucléophile dans le cadre de la définition des substituants, ou bien
(b) on isomérise thermiquement ("par pyrolyse") des aryliminohétérocycles de formule générale (III) 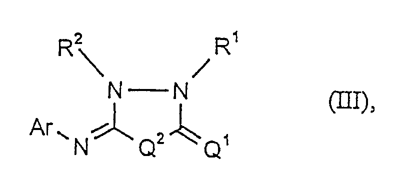
dans laquelle
Q¹, Q², R¹, R² et Ar ont les définitions indiquées ci-dessus,
éventuellement en présence d'un auxiliaire de réaction et en présence éventuelle d'un diluant.

4. Compositions herbicides, **caractérisées par** une teneur en au moins un N-aryl-hétérocycle azoté suivant la revendication 1 ou 2.

5. Procédé pour combattre des plantes indésirables, **caractérisé en ce qu'**on fait agir des N-aryl-hétérocycles azotés suivant la revendication 1 ou 2 sur les plantes indésirables et/ou sur leur milieu.

6. Utilisation de N-aryl-hétérocycles azotés suivant la revendication 1 ou 2 pour combattre des plantes indésirables.

7. Procédé de préparation de compositions herbicides, **caractérisé en ce qu'**on mélange des N-aryl-hétérocycles azotés suivant la revendication 1 ou 2 avec des diluants et/ou des agents tensio-actifs.
